(19) Europäisches Patentamt
European Patent Office
Office européen des brevets

(11) Veröffentlichungsnummer: **0 108 708**
**B1**

(12) **EUROPÄISCHE PATENTSCHRIFT**

(45) Veröffentlichungstag der Patentschrift: **14.06.89**

(21) Anmeldenummer: **83810443.8**

(22) Anmeldetag: **03.10.83**

(51) Int. Cl.⁴: **A 01 N 47/36**, C 07 D 251/16, C 07 D 251/18, C 07 D 251/20, C 07 D 251/22, C 07 D 251/24, C 07 D 239/46, C 07 D 239/42

(54) **N-(Cyclopropyl-triazinyl- und -pyrimidinyl)-N'-(arylsulfonyl)harnstoffe mit herbizider Wirkung.**

(30) Priorität: **06.10.82 CH 5874/82**

(43) Veröffentlichungstag der Anmeldung: **16.05.84 Patentblatt 84/20**

(45) Bekanntmachung des Hinweises auf die Patenterteilung: **14.06.89 Patentblatt 89/24**

(84) Benannte Vertragsstaaten: **AT BE CH DE FR GB IT LI LU NL SE**

(56) Entgegenhaltungen:
**EP-A- 0 030 141**
**EP-A- 0 044 807**
**EP-A- 0 056 969**
**US-A- 3 441 560**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Klybeckstrasse 141, CH-4002 Basel (CH)**

(72) Erfinder: **Szczepanski, Henry, Dr., Bodenmatt, CH-4323 Wallbach (CH)**

**EP 0 108 708 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft neue, herbizid wirksame N-(Cyclopropyl-triazinyl- und -pyrimidinyl)-N'-(arylsulfonyl)harnstoffe, Verfahren zu ihrer Herstellung, sie als Wirkstoffe enthaltende Mittel, sowie deren Verwendung zum Bekämpfen von Unkräutern vor allem selektiv in Nutzpflanzenkulturen oder zum Regulieren des Pflanzenwachstums.

Die erfindungsgemäßen N-(Cyclopropyl-triazinyl- und pyrimidinyl)-N'-(arylsulfonyl)harnstoffe entsprechen der allgemeinen Formel I

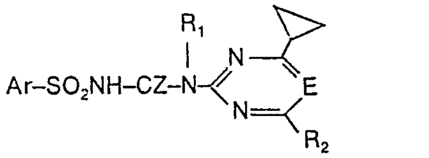

(I)

worin

Ar einen Phenylrest

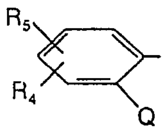

oder einen Naphthylrest

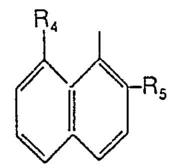

und

Q einen Rest X–A oder $R_3$,

A einen $C_3$–$C_6$-Alkinylrest, einen gegebenenfalls durch Halogen, $C_1$–$C_4$-Alkoxy, $C_1$–$C_4$-Alkylthio, $C_1$–$C_4$-Alkylsulfinyl, $C_1$–$C_4$-Alkylsulfonyl, $C_1$–$C_4$-Halogenalkoxy, $C_1$–$C_4$-Halogenalkylthio, $C_1$–$C_4$-Halogenalkylsulfinyl oder $C_1$–$C_4$-Halogenalkylsulfonyl substituierten $C_1$–$C_6$-Alkylrest oder einen gegebenenfalls durch die aufgezählten Reste substituierten $C_2$–$C_6$-Alkenylrest, einen Phenyl- oder Benzylrest,

E die Methingruppe oder Stickstoff,

X Sauerstoff, Schwefel, eine Sulfinyl- oder Sulfonylbrücke,

Z Sauerstoff oder Schwefel,

$R_1$ Wasserstoff, $C_1$–$C_4$-Alkyl oder $C_1$–$C_4$-Alkoxy,

$R_2$ Halogen, $C_1$–$C_3$-Alkyl, $C_1$–$C_3$-Halogenalkyl, $C_1$–$C_3$-Alkoxy, $C_1$–$C_3$-Halogenalkoxy, Amino, $C_1$–$C_3$-Alkylamino, Di-($C_1$–$C_3$-Alkyl)amino, $C_3$–$C_6$-Cycloalkyl oder $C_2$–$C_6$-Alkoxyalkyl,

$R_3$ Wasserstoff, Halogen, $C_1$–$C_5$-Alkyl, $C_2$–$C_5$-Alkenyl, $C_1$–$C_4$-Halogenalkyl, oder einen Rest $-X-R_6$, $-COZR_{11}$, $-NO_2$ oder $-CO-NR_8R_9$, $-CN$, $-COR_{10}$, $-NR_1R_7$, oder $-NR_1-COR_{12}$,

$R_4$ Wasserstoff, $C_1$–$C_4$-Alkyl, $C_1$–$C_4$-Alkoxy, $C_1$–$C_4$-Alkylthio, $C_1$–$C_4$-Halogenalkyl, $C_1$–$C_4$-Halogenalkoxy, Halogen oder Alkoxyalkyl mit höchstens 4 Kohlenstoffatomen,

$R_5$ dasselbe wie $R_3$ aber unabhängig davon,

$R_6$ und $R_7$ je $C_1$–$C_5$-Alkyl, $C_2$–$C_5$-Alkenyl oder $C_2$–$C_6$-Alkinyl,

$R_8$ und $R_9$ unabhängig voneinander Wasserstoff, $C_1$–$C_5$-Alkyl, $C_2$–$C_5$-Alkenyl oder $C_2$–$C_6$-Alkinyl,

$R_{10}$ Wasserstoff, $C_1$–$C_4$-Alkyl oder $C_1$–$C_3$-Halogenalkyl,

$R_{11}$ Wasserstoff, $C_1$–$C_4$-Alkyl, $C_1$–$C_3$-Halogenalkyl, $C_3$–$C_5$-Alkenyl, $C_3$–$C_5$-Alkinyl, Phenyl oder Benzyl und

$R_{12}$ dasselbe wie $R_1$ aber unabhängig davon bedeuten. Erfindungsgemäß sind auch die Salze dieser Verbindungen.

Harnstoffverbindungen, Triazinverbindungen und Pyrimidinverbindungen mit herbizider Wirkung sind allgemein bekannt. Kürzlich wurden Arylsulfamoyl-heterocyclyl-aminocarbamoylverbindungen mit herbizider und pflanzenwuchsregulierender Wirkung, beispielsweise in den europäischen Patentpublikationen Nr. 9419 und 10 560 beschrieben.

In den Definitionen ist unter Alkyl geradkettiges oder verzweigtes Alkyl zu verstehen: z.B. Methyl, Äthyl, n-Propyl, i-Propyl, die vier isomeren Butyl, n-Amyl, i-Amyl, 2-Amyl, 3-Amyl, n-Hexyl oder i-Hexyl.

Unter Alkoxy ist zu verstehen: Methoxy, Äthoxy, n-Propyloxy, i-Propyloxy und die vier isomeren Butyloxyreste, insbesondere aber Methoxy, Äthoxy oder i-Propoxy.

Beispiele für Alkylthio sind Methylthio, Äthylthio, n-Propylthio, i-Propylthio und n-Butylthio, insbesondere aber Methylthio und Äthylthio.

Beispiele für Alkenylreste sind Vinyl, Allyl, Isopropenyl, 1-Propenyl, 1-Butenyl, 2-Butenyl, 3-Butenyl, 1-Isobutenyl, 2-Isobutenyl, 1-Pentenyl, 2-Pentenyl, 3-Pentenyl und 4-Pentenyl, insbesondere aber Vinyl, Allyl und 4-Pentenyl.

Beispiele für Alkylsulfinyl sind Methylsulfinyl, Äthylsulfinyl, n-Propylsulfinyl und n-Butylsulfinyl, insbesondere aber Methylsulfinyl und Äthylsulfinyl.

Beispiele für Alkylsulfonyl sind Methylsulfonyl, Äthylsulfonyl, n-Propylsulfonyl und n-Butylsulfonyl, insbesondere aber Methylsulfonyl und Äthylsulfonyl.

Unter Halogen in den Definitionen sowie in Halogenalkyl, -alkoxy, -alkylsulfinyl, -alkylsulfonyl und -alkylthio sind Fluor, Chlor und Brom, vorzugsweise jedoch Fluor und Chlor zu verstehen.

Entsprechend sind unter Halogenalkyl bzw. Halogenalkylteilen von oben definierten Substituenten beispielsweise zu verstehen: Chlormethyl, Fluormethyl, Difluormethyl, Trifluormethyl, 2-Chloräthyl, 2,2,2-Trifluoräthyl, 1,1,2,2-Tetrafluoräthyl, Pentafluoräthyl, 1,1,2-Trifluor-2-chloräthyl, 2,2,2-Trifluor-1,1-dichloräthyl, Pentachloräthyl, 3,3,3-Trifluorpropyl, 2,3-Dichlorpropyl, 1,1,2,3,3,3-Hexafluorpropyl, insbesondere aber Chlormethyl, Difluormethyl, Trifluormethyl und Trichlormethyl.

Alkinylresten in den Definitionen der obigen Symbole entsprechen in der Regel Propargyl, 2-Butinyl, 3-Butinyl, sowie isomere Pentinyl- oder Hexinylreste, vorzugsweise ist der Alkinylrest jedoch durch Propargyl oder 2- oder 3-Butinyl verkörpert.

Die Erfindung umfaßt ebenfalls die Salze, die die Verbindungen der allgemeinen Formel I mit Aminen, Alkali- und Erdalkalimetallbasen oder quaternären Ammoniumbasen bilden können.

Unter Alkali- und Erdalkalimetallhydroxiden als Salzbildner sind die Hydroxide von Lithium, Natrium, Kalium, Magnesium oder Calcium hervorzuheben, insbesondere aber die von Natrium oder Kalium.

Beispiele für zur Salzbildung geeignete Amine sind primäre, sekundäre und tertiäre aliphatische und aromatische Amine wie Methylamin, Äthylamin, Propylamin, i-Propylamin, die vier isomere Butylamine, Dimethylamin, Diäthylamin, Diäthanolamin, Dipropylamin, Diisopropylamin, Di-n-butylamin, Pyrrolidin, Piperidin, Morpholin, Trimethylamin, Triäthylamin, Tripropylamin, Chinuclidin, Pyridin, Chinolin und i-Chinolin, insbesondere aber Äthyl-, Propyl-, Diäthyl- oder Triäthylamin, vor allem aber iso-Propylamin und Diäthanolamin.

Beispiele für quaternäre Ammoniumbasen sind im allgemeinen die Kationen von Halogenammoniumsalzen, z.B. das Tetramethylammoniumkation, das Trimethylbenzylammoniumkation, das Triäthylbenzylammoniumkation, das Tetraäthylammoniumkation, das Trimethyläthylammoniumkation, aber auch das Ammoniumkation.

Von den erfindungsgemäßen Verbindungen der allgemeinen Formel I sind die Verbindungen bevorzugt, in denen

– Ar einen in ortho-Stellung durch Q substituierten Phenylrest, E das Stickstoffatom oder die Methingruppe, $R_1$ Wasserstoff oder Methyl, $R_2$ einen $C_1$–$C_3$-Alkyl-, $C_1$–$C_3$-Halogenalkyl-, $C_1$–$C_3$-Alkoxy, $C_1$–$C_3$-Halogenalkoxy- oder $C_1$–$C_3$-Alkylaminorest und Z Sauerstoff bedeuten.

– Ar einen in ortho-Stellung durch $C_1$–$C_4$-Alkoxycarbonyl substituierten Phenylrest, E das Stickstoffatom oder die Methingruppe, $R_1$ Wasserstoff oder Methyl, $R_2$ einen $C_1$–$C_3$-Alkyl, $C_1$–$C_3$-Haloalkyl-, $C_1$–$C_3$-Alkoxy-, $C_1$–$C_3$-Haloalkoxy- oder $C_1$–$C_3$-Alkylaminorest und Z Sauerstoff bedeuten.

– Ar einen in ortho-Stellung durch $C_1$–$C_3$-Haloalkoxy substituierten Phenylrest, E das Stickstoffatom oder die Methingruppe, $R_1$ Wasserstoff oder Methyl, $R_2$ einen $C_1$–$C_3$-Alkyl-, $C_1$–$C_3$-Haloalkyl-, $C_1$–$C_3$-Alkoxy-, $C_1$–$C_3$-Haloalkoxy- oder $C_1$–$C_3$-Alkylaminorest und Z Sauerstoff bedeuten.

– Ar einen in ortho-Stellung durch Halogen oder Nitro substituierten Phenylrest, E das Stickstoffatom oder die Methingruppe, $R_1$ Wasserstoff oder Methyl, $R_2$ einen $C_1$–$C_3$-Alkyl-, $C_1$–$C_3$-Haloalkyl-, $C_1$–$C_3$-Alkoxy-, $C_1$–$C_3$-Haloalkoxy- oder $C_1$–$C_3$-Alkylaminorest und Z Sauerstoff bedeuten.

– Speziell die Verbindungen

N-(4-Cyclopropyl-6-methyl-pyrimidin-2-yl)-N'-(2-methoxycarbonylbenzolsulfonyl)-harnstoff,

N-(4-Cyclopropyl-6-methoxy-1,3,5-triazin-2-yl)-N'-(2-methoxycarbonylbenzolsulfonyl)-harnstoff,

N-(4-Cyclopropyl-6-äthoxy-1,3,5-triazin-2-yl)-N'-(2-difluormethoxybenzolsulfonyl)-harnstoff,

N-(4-Cyclopropyl-6-äthoxy-1,3,5-triazin-2-yl)-N'-(2-methoxycarbonylbenzolsulfonyl)-harnstoff.

Die Herstellung der Verbindungen der allgemeinen Formel I erfolgt in einem inerten, organischen Lösungsmittel.

Nach einem ersten Verfahren werden die Verbindungen der allgemeinen Formel I erhalten, indem man ein Sulfonamid der Formel II

$$Ar–SO_2NH_2 \quad (II),$$

worin Ar die unter allgemeiner Formel I gegebenen Bedeutung hat, in Gegenwart einer Base mit einem N-Pyrimidinyl- oder -Triazinylcarbamat der Formel III

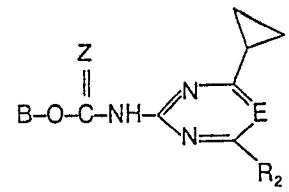

(III),

worin E, $R_2$ und Z die unter allgemeiner Formel I gegebene Bedeutung haben und B–O– einen abspaltbaren Phenoxy-, Phenylalkoxy- oder Alkoxyrest bedeutet, umsetzt.

Nach einem zweiten Verfahren gelangt man zu Verbindungen der allgemeinen Formel I, worin R Wasserstoff bedeutet, indem man ein Sulfonylisocyanat oder -isothiocyanat der Formel IV

$$Ar–SO_2–N=C=Z \quad (IV),$$

worin Ar und Z die unter allgemeiner Formel I gegebene Bedeutung haben, gegebenenfalls in Gegenwart einer Base, mit einem Amin der Formel V

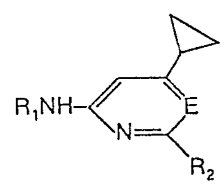

(V),

worin E, $R_1$ und $R_2$ die unter allgemeiner Formel I gegebene Bedeutung haben, umsetzt.

Nach einem weiteren Verfahren werden die Verbindungen der allgemeinen Formel I hergestellt, indem man ein Sulfonamid der oben angegebenen Formel II gegebenenfalls in Gegenwart einer Base mit einem Isocyanat oder Isothiocyanat der Formel VI

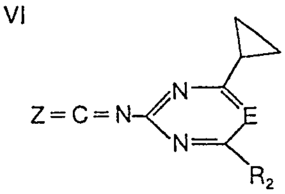

(VI),

worin

E, $R_2$ und Z die unter allgemeiner Formel I gegebene Bedeutung haben, umsetzt.

Weiterhin kann man die Verbindungen der allgemeinen Formel I auch erhalten, indem man ein N-Phenylsulfonylcarbamat der Formel VII

$$Ar–SO_2NHCOO–B \quad (VII),$$

worin Ar und B die oben gegebene Bedeutung haben, mit einem Amin der oben angegebenen Formel V umsetzt.

Die so erhaltenen Harnstoffe der allgemeinen Formel I können gewünschtenfalls mittels Aminen, Alkalimetall- oder Erdalkalimetallhydroxiden oder quaternären Ammoniumbasen in Additionssalze übergeführt werden. Dieses geschieht beispielsweise durch Umsetzen mit der äquimolaren Menge Base und Verdampfen des Lösungsmittels.

Die Ausgangsstoffe der Formeln II, IV, und VII sind bekannt oder können nach bekannten Methoden hergestellt werden.

Die Cyclopropyl-pyrimidine und Cyclopropyltriazine der Formel V, denen auch die Zwischenprodukte der Formeln III und VI zugrunde liegen sind z.T. neu. Sie und ihre Herstellung ist ebenfalls ein Teil dieser Erfindung.

2-Amino-4-cyclopropyl-6-methyl-pyrimidin wird hergestellt, indem man Cyclopropancarbonsäure oder deren Anhydrid in Aceton mit Bortrifluorid-Gas behandelt. Es entsteht Cyclopropylbutan-1,3-dion welches isoliert und dann in wäßriger oder alkoholischer Lösung mit Guanidin oder einem Guanidin-Salz kondensiert wird, entsprechend dem Schema

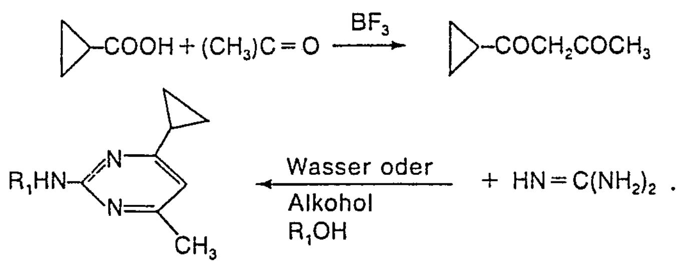


Diese Verbindung kann direkt mit einem Arylsulfonylisocyanat umgesetzt werden oder sie kann vor dem Umsetzen in bekannter Weise in ein Isocyanat oder einen Carbaminsäureester umgewandelt werden.

2-Amino-4-cyclopropyl-6-hydroxy-pyrimidin wird erhalten durch Umsetzung eines Cyclopropylcarbonsäurehalogenides, z.B. des Chlorides oder Bromides mit 2,2-Dimethyl-4,6-dioxo-dioxan (Malonsäure-acetonacetal) in einem basischen Lösungsmittel, wie z.B. in Pyridin. Es entsteht dabei 2,2-Dimethyl-4,6-dioxy-5-cyclopropylcarbonyl-dioxan, welches in siedendem Alkohol oder in Wasser zur Cyclopropyloxo-essigsäure respektive einem Ester davon gespalten wird. Diese letztere Verbindung wird mit Guanidin in Wasser oder einem niederen Alkohol zum 2-Amino-4-cyclopropyl-6-hydroxy-pyrimidin umgesetzt entsprechend dem Schema

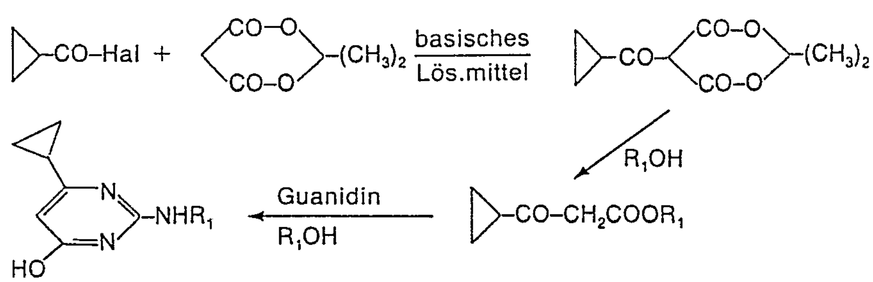


Die Hydroxygruppe kann durch ein Halogenatom ersetzt werden, wenn man diese Verbindung mit einem Halogenierungsmittel wie z.B. Phosphoroxychlorid oder -bromid behandelt. Das Halogenatom kann seinerseits in bekannter Weise durch andere Substituenten $R_2$ ersetzt werden und die so erhaltenen 2-Amino-4-cyclopropylpyrimidine können direkt mit einem Arylsulfonylisocyanat umgesetzt werden oder sie können vor dem Umsetzen in bekannter Weise in ein Isocyanat oder einen Carbaminsäureester umgewandelt werden.

2-Amino-4-cyclopropyl-6-trichlormethyl-1,3,5-triazin wird hergestellt, indem man 2 Mol Trichloracetonitril mit einem Mol Cyclopropylnitril in Gegenwart von Chlorwasserstoff zum 4-Cyclopropyl-2,6-bis-(trichlormethyl)-1,3,5-triazin kondensiert und diese Verbindung dann mit einem Mol Ammoniak oder einem Amin bei Temperaturen von 0–160 °C unter Normaldruck umsetzt, entsprechend dem Schema

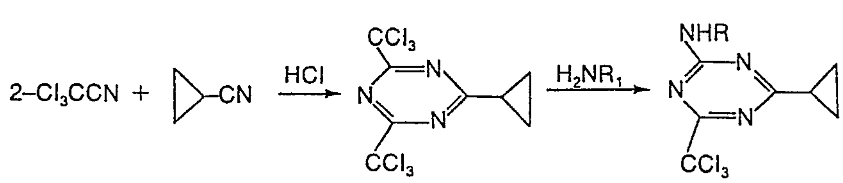

Durch Umsetzen entweder des 4-Cyclopropyl-2,6-bis-trichlormethyl-1,3,5-triazin oder des 2-Amino-4-cyclopropyl-6-trichlormethyl-1,3,5-triazin in einem absoluten organischen Lösungsmittel, mit der mindestens molaren Menge eines Alkanols $R_2OH$ oder dessen Alkali-Salz kommt man zu den entsprechenden 2-Amino-4-cyclopropyl-6-alkoxy-1,3,5-triazinen der Formel V oder 2-Cyclopropyl-4,6-bis-(alkoxy)-1,3,5-triazinen, die beispielsweise bei höheren Temperaturen von 40–140 °C und unter Druck mit mindestens der molaren Menge Amin zu 2-Amino-4-cyclopropyl-6-alkoxy-1,3,5-triazin umgewandelt werden können. Die so erhaltenen 2-Amino-4-cyclopropyl-1,3,5-triazine können direkt mit einem Arylsulfonylisocyanat umgesetzt werden oder sie können durch Umsetzen in bekannter Weise in ein Isocyanat oder einen Carbaminsäureester umgewandelt werden.

2-(γ-Chlorpropyl)-4,6-bis-(trichlormethyl)-1,3,5-triazin

wird in analoger Weise durch Kondensation von 2 Mol Trichloracetonitril und einem Mol γ-Chlorbutyronitril in Gegenwart von Chlorwasserstoff erhalten. Diese Verbindung läßt sich mit der molaren Menge Amin $H_2NR_1$ bei 0–160 °C unter Normaldruck zum 2-Amino-4-(γ-chlorpropyl)-6-trichlormethyl-1,3,5-triazin umsetzen, welches mit einem Alkali-Alkoholat in einem geeigneten Lösungsmittel bei 0–200 °C in 2-Amino-4-cyclopropyl-6-alkoxy-1,3,5-triazin übergeht und dann mit einem Arylsulfonsäureisocyanat oder -carbaminsäurerest zum gewünschten Harnstoff der allgemeinen Formel I weiter verarbeitet wird.

Diese Umsetzungen zu Verbindungen der allgemeinen Formel I werden vorteilhafterweise in aprotischen, inerten, organischen Lösungsmitteln vorgenommen wie Methylenchlorid, Tetrahydrofuran, Acetonitril, Dioxan, Toluol.

Die Reaktionstemperaturen liegen vorzugsweise zwischen −20 und +120 °C. Die Umsetzungen verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten können ebenfalls durch Zugabe einiger Tropfen Base oder Isocyanat als Reaktionskatalysator verkürzt werden.

Die Endprodukte können durch Einengen und/oder Verdampfen des Lösungsmittels isoliert und durch Umkristallisieren oder Zerreiben des festen Rückstandes in Lösungsmitteln in denen sie sich nicht gut lösen, wie Äther, aromatischen Kohlenwasserstoffen oder chlorierten Kohlenwasserstoffen gereinigt werden.

Die Wirkstoffe der allgemeinen Formel I sind stabile Verbindungen. Ihre Handhabung bedarf keiner vorsorglichen Maßnahmen.

Bei geringen Aufwandmengen, die in der Regel bei 0,01 bis 1 kg pro Hektar liegen, zeichnen sich die Verbindungen der allgemeinen Formel I durch gute selektiv-wuchshemmende und selektivherbizide Eigenschaften aus, die sie ausgezeichnet zum Einsatz in Kulturen von Nutzpflanzen, insbesondere in Getreide, Baumwolle, Soja, Mais, Raps und Reis, befähigen. Es werden dabei teilweise auch Unkräuter geschädigt, welchen bisher nur mit Totalherbiziden beizukommen war.

Die Wirkungsart dieser Wirkstoffe ist unüblich. Viele sind translozierbar, d.h. sie werden von der Pflanze aufgenommen und an andere Stellen transportiert wo sie dann zur Wirkung kommen. So gelingt es beispielsweise, durch Oberflächenbehandlung perennierende Unkräuter bis in die Wurzeln zu schädigen. Die neuen Verbindungen der allgemeinen Formel I wirken bereits bei – im Vergleich zu anderen Herbiziden und Wuchsregulatoren – sehr geringen Aufwandmengen.

Die Verbindungen der allgemeinen Formel I haben außerdem starke pflanzenwuchsregulierende, insbesondere pflanzenwuchshemmende, Eigenschaften. Es werden sowohl Monokotyledonen als auch Dikotyledonen in ihrem Wachstum beeinträchtigt.

So können z.B. die in der Landwirtschaft in tropischen Gegenden häufig als «cover crops» (Bodenbedecker) angepflanzten Leguminosen durch die Verbindungen der allgemeinen Formel I in ihrem Wachstum selektiv gehemmt werden, so daß zwar die Bodenerosion zwischen den Kulturpflanzen verhindert wird, die «cover crops» jedoch nicht zur Konkurrenz für die Kultur werden können.

Weiter eignen sich die Verbindungen der allgemeinen Formel I um das Keimen von eingelagerten Kartoffeln zu verhindern. Bei Kartoffeln entwickeln sich bei der Einlagerung über den Winter häufig Keime, die Schrumpfen, Gewichtsverlust und Faulen zu Folge haben.

Bei größeren Aufwandmengen werden alle getesteten Pflanzen in ihrer Entwicklung so geschädigt, daß sie absterben.

Die Erfindung betrifft auch herbizide und pflanzenwachstumsregulierende Mittel, welche einen neuen Wirkstoff der allgemeinen Formel I enthalten, sowie Verfahren zur pre- und post-emergenten Unkrautbekämpfung und zur Hemmung des Pflanzenwuchses von monokotylen und dikotylen Pflanzen, insbesondere Gräsern, tropischen Bodenbedeckern und Tabakgeiztrieben.

Die Verbindungen der allgemeinen Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Gießen werden gleich wie die Art der Mittel den angestrebten Zielen und den gegebenen Verhältnissen entsprechend gewählt.

Die Formulierungen, d.h. die den Wirkstoff der allgemeinen Formel I und gegebenenfalls einen festen oder flüssigen Zusatzstoff enthaltenden Mittel, Zubereitungen oder Zusammensetzungen

werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen: aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dicotylphthalat, aliphatische Kohlenwasserstoffe wie Cyclohexan oder Paraffine, Alkohole und Glykole sowie deren Äther und Ester, wie Äthanol, Äthylenglykol, Äthylenglykolmonomethyl- oder äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnußöl oder Sojaöl; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z. B. Bimsstein, Ziegelbruch, Sepiolit oder Bentonit, als nicht sorptive Trägermaterialien z. B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der allgemeinen Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$–$C_{22}$), wie z. B. die Na- oder K-Salze der Öl- oder Stearinsäure, oder von natürlichen Fettsäuregemischen, die z. B. aus Kokosnuß- oder Talgöl gewonnen werden können, genannt. Ferner sind auch die Fettsäure-methyltaurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschließt, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Äthylenoxid-Addukten. Die sulfonierten Benzimidazolderivate enthalten vorzugsweise 2 Sulfonsäuregruppen und einen Fettsäurerest mit 8–22 C-Atomen. Alkylarylsulfonate sind z. B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z. B. Salze des Phosphorsäureesters eines p-Nonylphenol-(4–14)-äthylenoxid-Adduktes in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Äthylenglykoläthergruppen und 10 bis 100 Propylenglykoläthergruppen enthaltenden Polyäthylenoxidaddukte an Polypropylenglykol, Äthylendiaminopolypropylenglykol und Alkylpolypropylenglykol mit 1 bis 10 Kohlenstoffatomen in der Alkylkette. Die genannten Verbindungen enthalten üblicherweise pro Propylenglykoleinheit 1 bis 5 Äthylenglykoleinheiten.

Als Beispiele nichtionischer Tenside seien Nonylphenolpolyäthoxyäthanole, Ricinusölpolyglykoläther, Polypropylenpolyäthylenoxidaddukte, Tributylphenoxypolyoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige gegebenenfalls halogenierte Alkyl-, Benzyl oder niedrige Hydroxyalkylreste aufweisen. Die Salze liegen vorzugsweise als Halogenide, Methylsulfate oder Äthylsulfate vor, z. B. das Stearyltrimethylammoniumchlorid oder das Benzyldi-(2-chloräthyl)-äthylammoniumbromid.

Die in der Formulierungstechnik gebräuchlichen Tenside sind u. a. in folgenden Publikationen beschrieben:

«Mc Cutcheon's Detergents and Emulsifiers Annual» MC Publishing Corp., Ringwood, New Jersey, 1979; Sisely and Wood, «Encyclopedia of Surface Active Agents», Chemical Publishing Co. Inc., New York, 1964.

Solche pestiziden Zubereitungen enthalten in der Regel 0,1 bis 95%, insbesondere 0,1 bis 80%, Wirkstoff der allgemeinen Formel I, 1 bis 99,9% eines festen oder flüssigen Zusatzstoffes und 0 bis 25%, insbesondere 0,1 bis 25% eines Tensides.

Insbesondere setzen sich bevorzugte Formulierungen folgendermaßen zusammen: (% = Gewichtsprozent).

| | |
|---|---|
| Emulgierbare Konzentrate | |
| Aktiver Wirkstoff: | 1 bis 20%, bevorzugt 5 bis 10% |
| oberflächenaktives Mittel: | 5 bis 30%, vorzugsweise 10 bis 20% |
| flüssiges Trägermittel: | 50 bis 94%, vorzugsweise 70 bis 85%. |
| Stäube | |
| Aktiver Wirkstoff: | 0,1 bis 10%, vorzugsweise 0,1 bis 1% |
| festes Trägermittel: | 99,9 bis 90%, vorzugsweise 99,9 bis 99%. |
| Suspension-Konzentrate | |
| Aktiver Wirkstoff: | 5 bis 75%, vorzugsweise 10 bis 50% |
| Wasser: | 94 bis 25%, vorzugsweise 90 bis 30% |
| oberflächenaktives Mittel: | 1 bis 40%, vorzugsweise 2 bis 30%. |
| Benetzbare Pulver | |
| Aktiver Wirkstoff: | 0,5 bis 90%, vorzugsweise 1 bis 80% |
| oberflächenaktives Mittel: | 0,5 bis 20%, vorzugsweise 1 bis 15% |
| festes Trägermittel: | 5 bis 95%, vorzugsweise 15 bis 90%. |
| Granulate | |
| Aktiver Wirkstoff: | 0,5 bis 30%, vorzugsweise 3 bis 15% |
| festes Trägermittel: | 99,5 bis 70%, vorzugsweise 97 bis 85%. |

Während als Handelsware eher konzentrierte Mittel bevorzugt werden, verwendet der Endverbraucher in der Regel verdünnte Mittel. Die Anwendungsformen können bis hinab zu 0,001% an Wirkstoff verdünnt werden. Die Aufwandmengen betragen in der Regel 0,001 bis 10 kg AS/ha, vorzugsweise 0,025 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

In den folgenden Beispielen sind die Temperaturen in Celsiusgraden °C, die Drücke in Millibar mb angegeben.

Beispiel 1:
Herstellung von 2-Cyclopropyl-4,6-bis-trichlormethyl-1,3,5-triazin (Zwischenprodukt).

Man kühlt 760 g Trichloracetonitril auf −15 °C ab und bläst Chlorwasserstoff-Gas durch die Lösung bis zur Sättigung. Dann gibt man langsam unter Kühlung und Chlorwasserstoffgas-Zugabe 230 g Cyclopropylnitril zu, so daß die Temperatur nicht über −10 °C steigt. Dann wird das Kühlbad weggenommen und das Reaktionsgemisch bei Raumtemperatur gerührt, bis sich die Temperatur auf 15 °C erhöht. Ab 10 °C setzt mit mäßig exothermer Reaktion Wasserstoff-Abspaltung ein, welche nach 20 Stunden Rühren bei 15–20 °C schließlich beendet ist.

Man gibt nun je 1,5 l Äther und Hexan zum Reaktionsgemisch, rührt das Ganze gut durch und filtriert. Das Filtrat wird eingeengt, der Rückstand in 700 ml Hexan aufgekocht, nochmals filtriert und das Filtrat erneut eingedampft. Der Rückstand wird aus Methanol umkristallisiert. Man erhält 350 g des obigen Triazins vom Schmelzpunkt 100–102 °C.

Beispiel 2:
Herstellung von 2-Amino-4-cyclopropyl-6-trichlormethyl-1,3,5-triazin.

Man löst 71,2 g 2-Cyclopropyl-4,6-bis-trichlormethyl-1,3,5-triazin in 70 ml Tetrahydrofuran und gibt bei Raumtemperatur unter Rühren 300 ml konzentrierte wäßrige Ammoniaklösung zu. Man rührt während 30 Minuten, verdünnt dann mit Wasser und extrahiert die entstandene Emulsion mit Äther. Die Ätherphasen werden gesammelt, getrocknet, filtriert und eingedampft. Der Rückstand kristallisiert. Es verbleiben 48 g kristallines Titelprodukt vom Schmelzpunkt 111–114 °C.

Beispiel 3:
Herstellung von 2-Cyclopropyl-4-methoxy-6-trichlormethyl-1,3,5-triazin.

Zu einer Lösung von 48 g 2-Cyclopropyl-4,6-bis-(trichlormethyl)-1,3,5-triazin in 200 ml MeOH gibt man unter Rühren 4,9 g Natrium-Methylat. Nach 1 Stunde Rühren bei Raumtemperatur wird filtriert und das Filtrat zur Trockne eingedampft.

Man erhält 28,5 g Titelverbindung als hellrotes Öl welches nach einiger Zeit erstarrt. Schmelzpunkt 49–51 °C.

Beispiel 4:
Herstellung von 2-Cyclopropyl-4,6-dimethoxy-1,3,5-triazin (Zwischenprodukt).

Man gibt zu einer Lösung von 71,2 g 2-Cyclopropyl-4,6-bis-(trichlormethyl)-1,3,5-triazin in 200 ml Methanol 8,1 g Natrium-Methylat und rührt während 5 Stunden bei Raumtemperatur. Dann wird die Lösung zur Trockne eingedampft und der

Rückstand mit 200 ml Äther verrührt. Die erhaltene Suspension wird filtriert und das Filtrat eingeengt. Der Rückstand kristallisiert und wird aus Äther/Hexan nochmals umkristallisiert. Man erhält so 35,7 g des obigen Triazins vom Schmelzpunkt 68–70 °C.

Beispiel 5:
Herstellung von 2-Cyclopropyl-4-amino-6-methoxy-1,3,5-triazin (Zwischenprodukt).

Man gibt zu einer Lösung von 10,8 g Natrium-Methylat in 50 ml Methanol 28,9 g 2-Amino-4-(3-chlorpropyl)-6-trichlormethyl-1,3,5-triazin und rührt das Gemisch während 80 Min. bei 60 °C. Die entstandene Suspension wird dann abgenutscht, mit 250 ml Wasser verdünnt und 3 mal mit je 200 ml Äthylenchlorid extrahiert. Die organischen Phasen werden getrocknet, filtriert und eingedampft. Das zurückbleibende Öl wird über eine Kieselgel-Kolonne gereinigt. Als Laufmittel dient Methylenchlorid/Äther 3:1. Nach Verdampfen des Laufmittels verbleibt 2,1 g Titelprodukt, das aus Äther/Chloroform umkristallisiert wird. Schmelzpunkt 158–159 °C.

Beispiel 6:
Herstellung von 2-Amino-4-cyclopropyl-6-methoxy-1,3,5-triazin (Zwischenprodukt).

Man bläst durch eine Lösung von 5 g 2-Cyclopropyl-4,6-dimethoxy-1,3,5-triazin in 30 ml Methanol bei Raumtemperatur gasförmiges Ammoniak bis zur Sättigung. Die Lösung wird dann in einem Bombenrohr (Autoklav) bei 140 °C während einer Stunde gerührt. Nach dem Abkühlen filtriert man das Reaktionsgemisch. Man erhält so 0,5 g Titelprodukt als hellbraune Kristalle, welche bei 156–157 °C schmelzen.

Beispiel 7:
Herstellung von 2-Amino-4-cyclopropyl-6-methoxy-1,3,5-triazin (Zwischenprodukt).

Man versetzt eine Lösung von 1 g 2-Cyclopropyl-4-methoxy-6-trichlormethyl-1,3,5-triazin in 3 ml Tetrahydrofuran mit 5 ml konzentriertem wäßrigem Ammoniak, schließt das Ganze in ein Bombenrohr ein und erhitzt während 30 Minuten auf 80 °C. Nach dem Abkühlen wird filtriert, der Filterrückstand in 30 ml Methylenchlorid aufgelöst, über Magnesiumsulfat getrocknet und eingedampft. Der Rückstand kristallisiert. Es verbleiben 0,4 g weiße Kristalle, welche bei 157–158 °C schmelzen.

Beispiel 8:
Herstellung von 2-Amino-4-(3-chlorpropyl)-6-trichlormethyl-1,3,5-triazin (Zwischenprodukt).

Man löst 102,3 g 2-(3-Chlorpropyl)-4,6-bis-(trichlormethyl)-1,3,5-triazin in 100 ml Tetrahydrofuran und versetzt diese Lösung, während bei Raumtemperatur gerührt wird, mit 400 ml konzentrierter wäßriger Ammoniaklösung. Nach 30 Minuten gibt man 500 ml Wasser dazu und extrahiert das Reaktionsgemisch 2 mal mit je 100 ml Äther. Die Ätherphasen werden über Magnesiumsulfat getrocknet, filtriert und eingedämpft. Es verbleiben 68,3 g Titelprodukt als hellbraunes Öl.

Beispiel 9:
Herstellung von 2-(3-Chlorpropyl)-4,6-bis-(trichlormethyl)-1,3,5-triazin (Zwischenprodukt).

Man bläst bei einer Temperatur von −20 °C gasförmigen Chlorwasserstoff durch eine Lösung von 103 g 4-Chlorbutyronitril in 298 g Trichloracetonitril bis zur Sättigung. Dann wird langsam unter Rühren auf Raumtemperatur erwärmt, wobei nur eine relativ geringe Gasentwicklung stattfindet und ein kristalliner Niederschlag ausfällt. Man gibt noch einen Liter Toluol zu und rührt das Reaktionsgemisch bei 85 °C bis sich kein HCl-Gas mehr entwickelt. Man hört dann mit Rühren auf, läßt erkalten und dekantiert die klare Lösung vom Schlamm, der sich gesetzt hat. Die Lösung wird eingedampft und das verbleibende Öl im Hochvakuum destilliert. Man erhält so 294 g Titelverbindung. Siedepunkt 150–160 °C/0,2 mbar. Brechungsindex $n_D^{27}$: 1,5498.

Beispiel 10:
Herstellung von 2-Amino-4-cyclopropyl-6-methoxy-1,3,5-triazin (Zwischenprodukt).

Eine Lösung von 10,8 g Natriummethylat in 50 ml Methanol wird mit 25,3 g 2-Amino-4-cyclopropyl-6-trichlormethyl-1,3,5-triazin versetzt und 80 Minuten bei einer Temperatur von 60 °C gerührt. Anschließend gibt man 300 ml Wasser zu und filtriert die entstandene Suspension. Der Filterrückstand wird zweimal mit je 100 ml Acetylacetat aufgeschlämmt und erneut filtriert. Die organischen Phasen werden gesammelt, über Magnesiumsulfat getrocknet und eingedampft. Man erhält so ein Öl, das kristallisiert, wenn man es in Äther verrührt. Man erhält so 6,7 g Titelverbindung vom Schmelzpunkt 158–159 °C.

In analoger Weise zu diesen Beispielen werden folgende Triazin-Ausgangsprodukte hergestellt:

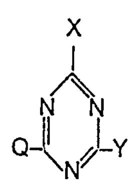

| Q | X | Y | phys. Daten, °C | |
|---|---|---|---|---|
| $CCl_3$ | Cyclopropyl | $CCl_3$ | Smp. 100–102° | Beisp. 1 |
| $NH_2$ | Cyclopropyl | $CCl_3$ | Smp. 114–116° | Beisp. 2 |
| $OCH_3$ | Cyclopropyl | $CCl_3$ | Smp. 49–51° | Beisp. 3 |
| $CH_3$ | Cyclopropyl | $CH_3$ | Smp. 68–70° | Beisp. 4 |

(Fortsezung)

| Q | X | Y | phys. Daten, °C | |
|---|---|---|---|---|
| $NH_2$ | Cyclopropyl | $OCH_3$ | Smp. 158–159° | Beisp. 5, 6, 7, 10 |
| $NH_2$ | Cl $C_3H_6$– | $CCl_3$ | Öl | Beisp. 8 |
| $CCl_3$ | Cl $C_3H_6$– | $CCl_3$ | Öl $n_D^{27}$: 1,5498 | Beisp. 9 |
| $NHC_3H_{7n}$ | Cyclopropyl | $CCl_3$ | Öl | |
| $NHC_3H_{7n}$ | Cyclopropyl | $NHC_2H_5$ | Öl | |
| $OCH_3$ | Cyclopropyl | OH | Smp. 160–164° | |
| $NHC_3H_{7n}$ | Cyclopropyl | $NH_2$ | Harz | |
| $OC_2H_5$ | Cyclopropyl | $CCl_3$ | Öl | |
| $NHCH_3$ | Cyclopropyl | $CCl_3$ | Smp. 100–102° | |
| $NHC_2H_5$ | Cyclopropyl | $CCl_3$ | Smp. 47–49° | |
| $NHC_3H_7$iso | Cyclopropyl | $CCl_3$ | Öl | |
| $N(CH_3)_2$ | Cyclopropyl | $CCl_3$ | Smp. 59–61° | |
| $NHCH_3$ | Cyclopropyl | $OCH_3$ | | |
| $NHCH_3$ | Cyclopropyl | $OC_2H_5$ | | |
| $NHCH_3$ | Cyclopropyl | $OCH_2CF_3$ | | |
| $NH_2$ | Cyclopropyl | $OC_2H_5$ | | |
| $NH_2$ | Cyclopropyl | $OCH_2CF_3$ | | |
| $NH_2$ | Cyclopropyl | $OC_2H_4Cl$ | | |
| $NH_2$ | Cyclopropyl | $OC_2H_4OCH_3$ | | |
| $NH_2$ | Cyclopropyl | $OCH(CH_3)_2$ | | |
| $NH_2$ | Cyclopropyl | $SCH_3$ | | |
| $NH_2$ | Cyclopropyl | $NHNHCH_3$ | | |
| $NH_2$ | Cyclopropyl | $N(CH_3)NHCH_3$ | | |
| $NH_2$ | Cyclopropyl | $NHCOCH_3$ | | |
| $NH_2$ | Cyclopropyl | $NH_2$ | | |

Beispiel 11:
Herstellung von 1-Cyclopropylbutan-1,3-dion (Zwischenprodukt).

Man leitet während einer Stunde bei 40 °C Bortrifluorid-Gas in ein Gemisch von 191 g Cyclopropancarbonsäure und 45 g Aceton. Es entsteht ein schwarzes öliges Produkt, das man mit 800 ml Äther verdünnt. Dann wird die Ätherphase 4 mal mit je 300 ml Wasser gewaschen und schließlich unter Eiskühlung mit 30%iger Natronlauge versetzt bis der pH-Wert ~9 erreicht ist. Die Ätherschicht wird abgetrennt und die wäßrige Phase noch dreimal mit je 300 ml Äther extrahiert. Die Ätherphasen werden gesammelt, über Natriumsulfat getrocknet, mit Aktivkohle gereinigt, filtriert und eingedampft. Das zurückbleibende Öl wird durch Chromatographie über eine Silikagel-Kolonne mit dem Laufmittel Äther/Hexan 1:3 gereinigt. Nach Verdampfen des Lösungsmittels verbleiben 22 g Titelverbindung als helles Öl. Brechungsindex $n_D^{24}$: 1,488.

Beispiel 12:
Herstellung von 2-Amino-4-cyclopropyl-6-methylpyrimidin (Zwischenprodukt).

Ein Gemisch von 10 g 1-Cyclopropyl-butan-1,3-dion und 150 g Wasser wird mit 15 g Guanidincarbonat versetzt und während 5 Stunden bei 95 °C gerührt. Dann wird bis auf 50 ml Volumen eingedampft und das abgekühlte wäßrige Konzentrat dreimal mit je 100 ml Äthylenchlorid extrahiert. Die organische Phase wird über Magnesiumsulfat getrocknet und eingedampft. Der Rückstand kristallisiert. Man erhält so 5 g Titelverbindung vom Schmelzpunkt 113–115 °C.

Beispiel 13:
Herstellung von N-(4-Cyclopropyl-6-methylpyrimidin-2-yl)-N'-(2'-methoxycarbonylbenzonsulfonyl)-harnstoff

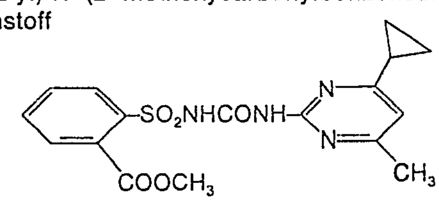

Man gibt 2,4 g 2-Methoxycarbonyl-benzolsulfonyl-isocyanat zu einem Gemisch von 1 g 2-Amino-4-cyclopropyl-6-methylpyridin in 7 ml Äther und 7 ml Äthylenchlorid und rührt bei Raumtemperatur während 14 Stunden. Der ausgefallene Niederschlag wird filtriert. Man erhält so 2,2 g des obigen Harnstoffes vom Schmelzpunkt 173–175 °C.

Beispiel 14:
Herstellung von N-(4-Cyclopropyl-6-methoxy-1,3,5-triazin-2-yl)-N'-(2-methoxycarbonylbenzolsulfonyl)-harnstoff

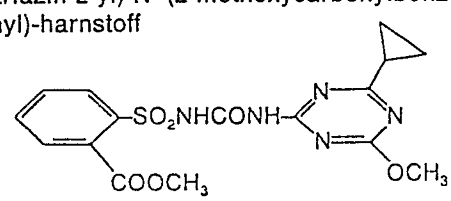

Man gibt unter Stickstoffatmosphäre und Rühren 2,1 g 2-Methoxycarbonylbenzolsulfonylisocyanat zu einer Lösung von 1,3 g 2-Amino-4-cyclopropyl-6-methoxy-1,3,5-triazin in 10 ml $CH_2Cl_2$. Es entsteht eine leicht exotherme Reaktion, die aber bald abklingt. Man rührt 14 Stunden bei Raumtemperatur und bringt dann die Lösung durch Zugabe von Äther zur Kristallisation. Der Niederschlag wird abfiltriert und getrocknet. Man erhält so 2,3 g des obigen Harnstoffes, welcher bei 166–168 °C schmilzt.

Beispiel 15:
Herstellung von N-(4-Cyclopropyl-6-trichlormethyl-1,3,5-triazin-2-yl)-N'-(2-difluormethoxybenzolsulfonyl)-harnstoff

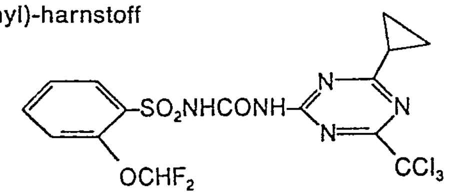

Man gibt 2,7 g 2-Difluoromethoxybenzolsulfonylisocyanat zu einer Lösung von 2,54 g 2-Amino-4-cyclopropyl-6-trichlormethyl-1,3,5-triazin und rührt das Ganze während 14 Stunden bei Raumtemperatur. Dann wird Hexan zugegeben bis das Reaktionsprodukt ausfällt. Man filtriert und trocknet den Filtrierrückstand. Man erhält so 3 g des obigen Harnstoffes, welcher bei 103–105 °C schmilzt.

Beispiel 16:
Herstellung von N-(4-Cyclopropyl-6-methoxy-1,3,5-triazin-2-yl)-N'-(2-difluormethoxybenzolsulfonyl)-harnstoff

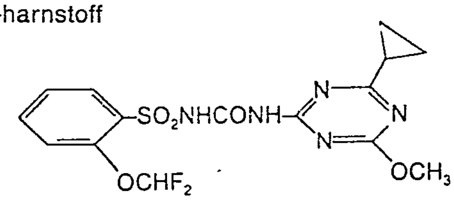

Man gibt 3,75 g 2-Difluormethoxybenzolsulfonylisocyanat zu einer Lösung von 2,5 g 2-Amino-4-cyclopropyl-6-methoxy-1,3,5-triazin in 15 ml Methylenchlorid und rührt während 70 Stunden bei Raumtemperatur. Dann wird eingeengt und der Rückstand mit 25 ml Äther versetzt. Es fällt ein kristalliner Niederschlag aus, der filtriert wird. Man erhält so 4,7 g des obigen Harnstoffes, welcher bei 121–124 °C schmilzt.

Beispiel 17:
Die Verbindung vom Beispiel 16 kann auch wie folgt hergestellt werden:

Man gibt eine Lösung von 0,54 g $NaOCH_3$ in 1,26 g Methanol zu einer Lösung von 0,9 g N-(4-Cyclopropyl-6-trichlormethyl-1,3,5-triazin-2-yl)-N'-(2-difluormethoxybenzolsulfonyl)-harnstoff (Beispiel 15) und rührt während einer Stunde bei 50 °C. Das Reaktionsgemisch wird dann in 100 ml Wasser gegossen, mit 10%iger wäßriger Salzsäure bis zum pH-Wert 1 angesäuert, viermal mit je 50 ml Methylenchlorid extrahiert. Die organischen Phasen werden über Magnesium getrocknet, filtriert und eingedampft. Es verbleibt ein Öl, welches nach Verreiben in Äther kristallisiert. Die Kristalle werden aus Äther umkristallisiert. Man erhält so 0,66 g des obigen Harnstoffes, welcher bei 122–124 °C schmilzt.

In analoger Weise zu diesen Beispielen werden folgende Sulfonylharnstoffe hergestellt:

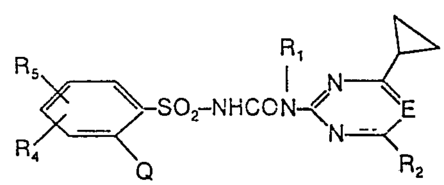

| No. | Q | $R_1$ | $R_2$ | E | Schmelzpunkt, °C |
|---|---|---|---|---|---|
| 1 | $COOCH_3$ | H | $OCH_3$ | N | 166–168 °, Beisp. 14 |
| 2 | $COOCH_3$ | H | $CCl_3$ | N | 162 °, Zers. |
| 3 | Cl | H | $OCH_3$ | N | 161–164 ° |
| 4 | $OCHF_2$ | H | $OCH_3$ | N | 121–124 °, Beisp. 16, 17 |
| 5 | $COOCH_3$ | H | $NHC_3H_{7n}$ | N | 187–188 ° |
| 6 | $NO_2$ | H | $OCH_3$ | N | 179–182 ° |
| 7 | $COOCH_3$ | H | $OC_2H_5$ | N | 154–156 ° |
| 8 | $OCH_3$ | H | $OCH_3$ | N | 142–146 ° |
| 9 | $OCHF_2$ | H | $CCl_3$ | N | 103–105 ° |
| 10 | $OCHF_2$ | H | $OC_2H_5$ | N | 143–144 ° |
| 11 | $NO_2$ | H | $OC_2H_5$ | N | 182–184 ° |
| 12 | $OCH_3$ | H | $OC_2H_5$ | N | 149–151 ° |
| 13 | $COOCH_3$ | $CH_3$ | $OCH_3$ | N | 149–151 ° |
| 14 | $OCHCF_2$ | $CH_3$ | $OCH_3$ | N | 118–120 ° |
| 15 | $OCH_3$ | $CH_3$ | $OCH_3$ | N | 121–123 ° |
| 16 | $COOCH_3$ | $CH_3$ | $CCl_3$ | N | 133–135 ° |
| 17 | $OCHF_2$ | H | $CCl_3$ | N | 138–140 °, Beisp. 15 |
| 18 | $OCHF_2$ | H | Cl | CH | |

(Fortsetzung)

| No. | Q | $R_1$ | $R_2$ | E | Schmelzpunkt, °C |
|---|---|---|---|---|---|
| 19 | $COOCH_3$ | H | $CH_3$ | CH | 173–175°, Beisp. 13 |
| 20 | $OCHF_2$ | H | $CH_3$ | CH | 168–169° |
| 21 | $OCH_3$ | H | $CH_3$ | CH | 160–162° |
| 22 | $NO_2$ | H | $CH_3$ | CH | 153–157° |
| 23 | $OCHF_2$ | H | $NHC_3H_7$iso | N | 233–236° |
| 24 | Cl | H | $NHC_3H_7$iso | N | 209–211° |
| 25 | $OC_2H_4OCH_3$ | H | $OCH_3$ | N | 130–132° |
| 26 | $COOCH_3$ | H | $NHC_3H_7$iso | N | 210–212° |
| 27 | $OCH_3$ | H | $NHC_3H_7$iso | N | 202–205° |
| 28 | $NO_2$ | H | $NHC_3H_7$iso | N | 211–215° |
| 29 | Cl | H | OH | CH | |
| 30 | $NO_2$ | H | OH | CH | |
| 31 | Cl | H | $CH_3$ | CH | |
| 32 | Cl | H | $OCH_3$ | CH | |
| 33 | Cl | H | $OC_2H_5$ | CH | |
| 34 | $OCH_3$ | H | $OCH_3$ | CH | |
| 35 | $OCH_3$ | H | $OC_2H_5$ | CH | |
| 36 | $OCH_3$ | H | $OCH_2CF_3$ | | |
| 37 | $NO_2$ | H | $OCH_3$ | CH | 201–203° |
| 38 | $NO_2$ | H | $OC_2H_5$ | CH | |
| 39 | $NO_2$ | H | $OCH_2CF_3$ | CH | |
| 40 | $OC_2H_4OCH_3$ | H | $CH_3$ | CH | |
| 41 | $COOCH_3$ | H | $OCH_3$ | CH | 176–178° |
| 42 | $COOCH_3$ | H | $OC_2H_5$ | CH | |
| 43 | $COOCH_3$ | H | $OCH_2CF_3$ | N | |
| 44 | $COOCH_3$ | H | $OCH_2CF_3$ | CH | |
| 45 | $NO_2$ | H | $OCH_2CF_3$ | N | |
| 46 | $OCHF_2$ | H | $OCH_2CF_3$ | N | |
| 47 | $OCHF_2$ | H | $OCH_3$ | CH | 170–172° |
| 48 | $OCHF_2$ | H | $OC_2H_5$ | CH | |
| 49 | $OCHF_2$ | H | $OCH_2CF_3$ | CH | |
| 50 | $OC_2H_4OCH_3$ | H | $OC_2H_5$ | N | |
| 51 | $OC_2H_4OCH_3$ | H | $OCH_2CF_3$ | N | |
| 52 | $OC_2H_4OCH_3$ | H | $CH_3$ | CH | |
| 53 | $OC_2H_4OCH_3$ | H | $OCH_3$ | CH | 178–181° |
| 54 | $OC_2H_4OCH_3$ | H | $OC_2H_5$ | CH | |
| 55 | $OC_2H_4OCH_3$ | H | $OCH_2CF_3$ | CH | |
| 56 | $SCHF_2$ | H | $OCH_3$ | N | |
| 57 | $SCHF_2$ | H | $OC_2H_5$ | N | |
| 58 | $SCHF_2$ | H | $OCH_2CF_3$ | N | |
| 59 | $SCHF_2$ | H | $CH_3$ | CH | |
| 60 | $SCHF_2$ | H | $OCH_3$ | CH | |
| 61 | $SCHF_2$ | H | $OC_2H_5$ | CH | |
| 62 | $SCHF_2$ | H | $OCH_2CF_3$ | CH | |
| 63 | $COOCH_3$ | H | Cl | CH | 190–193° |
| 64 | $COOCH_3$ | H | $NHC_2H_5$ | N | 208–210° |
| 65 | $OCHF_2$ | H | $NHC_2H_5$ | N | 230–232° |
| 66 | Cl | H | $N(CH_3)_2$ | N | 188–190° |
| 67 | $OCHF_2$ | H | $NHCH_3$ | N | 222–223° |
| 68 | $OCHF_2$ | H | $N(CH_3)_2$ | N | 160–162° |
| 69 | $COOCH_3$ | H | $N(CH_3)_2$ | N | 153–156° |
| 70 | Cl | H | $NCH_2H_5$ | N | 227–228° |
| 71 | $NO_2$ | H | $NHC_2H_5$ | N | 215–217° |
| 72 | Cl | H | $SCH_3$ | N | 120–125° |
| 73 | $NO_2$ | H | Cl | CH | 200° |
| 74 | $OC_2H_4OCH_3$ | H | Cl | CH | 153–154° |
| 75 | Cl | H | Cl | CH | 203–205° |
| 76 | OC(Cl) = CHCl | H | Cl | CH | 197–200° |
| 77 | OC(Cl) = CHCl | H | $OCH_3$ | CH | 212–215° |
| 78 | $CO_2CH_3$ | H | $CH_3$ | N | 158–160° |

(Fortsetzung)

| No. | Q | $R_1$ | $R_2$ | E | Schmelzpunkt, °C |
|---|---|---|---|---|---|
| 79 | $COOCH_3$ | H | $OCHF_2$ | N | 140–142° |
| 80 | $COOCH_3$ | H | $C_3H_5$(cyclo) | CH | 178–180° |
| 81 | $OCHF_2$ | H | $C_3H_5$(cyclo) | CH | 153–155° |
| 82 | Cl | H | $C_3H_5$(cyclo) | CH | 215–218° |
| 83 | F | H | $C_3H_5$(cyclo) | CH | 194–197° |
| 84 | $CH_3$ | H | $C_3H_5$(cyclo) | CH | 193–197° |
| 85 | OC(Cl)=CHCl | H | $C_3H_5$(cyclo) | CH | 214–215° |
| 86 | $OCHF_2$ | H | $CH_3$ | N | 152–153° |
| 87 | Cl | H | $CH_3$ | N | 155–157° |
| 88 | F | H | $CH_3$ | N | 159–161° |
| 89 | $OC_2H_4Cl$ | H | $CH_3$ | N | 163–165° |
| 90 | OC(Cl)=CHCl | H | $CH_3$ | N | 172–174° |
| 91 | $SCHF_2$ | H | $CH_3$ | N | 178–180° |
| 92 | $COOCH_3$ | H | $C_3H_5$(cyclo) | N | 186–188° |
| 93 | $OCHF_2$ | H | $C_3H_5$(cyclo) | N | 120–125° |
| 94 | Cl | H | $C_3H_5$(cyclo) | N | 195–197° |
| 95 | F | H | $C_3H_5$(cyclo) | N | 174–176° |
| 96 | $OCH_2CH_2Cl$ | H | $C_3H_5$(cyclo) | N | 178–180° |
| 97 | O(Cl)=CHCl | H | $C_3H_5$(cyclo) | N | 206–209° |
| 98 | $SCHF_2$ | H | $C_3H_5$(cyclo) | N | 165–168° |
| 99 | $OCHF_2$ | H | $CH_2OCH_3$ | N | 109–112° |
| 100 | Cl | H | $CH_2OCH_3$ | N | 172–175° |
| 101 | F | H | $CH_2OCH_3$ | N | 132–136° |
| 102 | $OCH_2CH_2Cl$ | H | $CH_2OCH_3$ | N | 142–146° |
| 103 | $OC_2H_4OCH_3$ | H | $CH_2OCH_3$ | N | 129–132° |
| 104 | $COOCH_3$ | H | $C_2H_5$ | N | 168–170° |
| 105 | $OCHF_2$ | H | $C_2H_5$ | N | 106–109° |
| 106 | Cl | H | $C_2H_5$ | N | 190–193° |
| 107 | F | H | $C_2H_5$ | N | 156–158° |
| 108 | $OCH_2CH_2Cl$ | H | $C_2H_5$ | N | 163–167° |
| 109 | $OCH_2CH_2Cl$ | H | $C_2H_5$ | N | 136–138° |
| 110 | $SCHF_2$ | H | $OCH_3$ | N | 163–165° |
| 111 | $OC_2H_4Cl$ | H | $OCH_3$ | N | 155–158° |
| 112 | Cl | H | $OC_2H_5$ | N | 139–142° |
| 113 | $OCH_2CH_2Cl$ | H | $OC_2H_5$ | N | 152–155° |
| 114 | $OCH_2CH_2OCH_3$ | H | $OC_2H_5$ | N | 132–135° |
| 115 | $SCHF_2$ | H | $OC_2H_5$ | N | 163–166° |
| 116 | $COOCH_3$ | H | $OC_2H_5$ | N | 136–139° |
| 117 | $COOCH_3$ | H | $SCH_3$ | CH | |
| 118 | $COOCH_3$ | H | $OC_2H_5$ | CH | |
| 119 | $OCF_3$ | H | $OCH_3$ | N | |
| 120 | $OCF_3$ | H | $OC_2H_5$ | N | |
| 121 | $OCHF_2$ | H | $SCH_3$ | CH | |
| 122 | $NO_2$ | H | $SCH_3$ | CH | |
| 123 | $OCH_2CH_2Cl$ | H | $SCH_3$ | CH | |
| 124 | $OCH_2CH_2Cl$ | H | $SCH_3$ | CH | |
| 125 | OC(Cl)=CHCl | H | $SCH_3$ | CH | |
| 126 | $SCH_2OCH_3$ | H | $OCH_3$ | N | |
| 127 | $SCH_2OCH_3$ | H | $OC_2H_5$ | N | |
| 128 | $SCH_2CH=CH_2$ | H | $OCH_3$ | N | |
| 129 | $SCH_2CH=CH_2$ | H | $OC_2H_5$ | N | |
| 130 | $SCH_2CH=CH_2$ | H | $OCH_3$ | CH | |
| 131 | $SCH_2CH=CH_2$ | H | $OC_2H_5$ | CH | |
| 132 | $SCH_2OCH_3$ | H | $OCH_3$ | CH | |
| 133 | $SCH_2OCH_3$ | H | $OC_2H_5$ | CH | |
| 134 | $SCH_2C\equiv CH$ | H | $OCH_3$ | N | |
| 135 | $SCH_2C\equiv CH$ | H | $OC_2H_5$ | N | |
| 136 | $SCH_2C\equiv CH$ | H | $OCH_3$ | CH | |
| 137 | $S(O)CH_2C\equiv CH$ | H | $OCH_3$ | N | |
| 138 | $S(O)CH_2\equiv CH$ | H | $OCH_3$ | CH | |
| 139 | $SO_2CH_2C\equiv CH$ | H | $OCH_3$ | N | |

(Fortsetzung)

| No. | Q | $R_1$ | $R_2$ | E | Schmelzpunkt, °C |
|---|---|---|---|---|---|
| 140 | $SO_2CH_2C\equiv CH$ | H | $OCH_3$ | CH | |
| 141 | $S(O)CH_2CH=CH_2$ | H | $OCH_3$ | N | |
| 142 | $OCH_2CH=CH_2$ | H | $OCH_3$ | N | |
| 143 | $OCH_2C\equiv CH$ | H | $OCH_3$ | N | |
| 144 | $NHCOCH_3$ | H | $OCH_3$ | N | |
| 145 | $CON(CH_3)_2$ | H | $OCH_3$ | N | |
| 146 | $SO_2CH_2C_6H_5$ | H | $OCH_3$ | N | |
| 147 | $SO_2CH_2C_6H_5$ | H | $OCH_3$ | CH | |
| 148 | $SO_2CH_2C_6H_5$ | H | $OC_2H_5$ | CH | |
| 149 | $SO_2CH_2C_6H_5$ | H | $OC_2H_5$ | N | |

Formulierungsbeispiele
Beispiel 18:
Formulierungsbeispiele für Wirkstoffe der allgemeinen Formel I (% = Gewichtsprozent)

| a) Spritzpulver | a) | b) | c) |
|---|---|---|---|
| Wirkstoff | 20% | 60% | 0,5% |
| Na-Ligninsulfonat | 5% | 5% | 5% |
| Na-Laurylsulfat | 3% | – | – |
| Na-Diisobutylnaphthalinsulfonat | – | 6% | 6% |
| Octylphenolpolyäthylenglykoläther (7–8 Mol AeO) | – | 2% | 2% |
| Hochdisperse Kieselsäure | 5% | 27% | 27% |
| Kaolin | 67% | – | – |
| Natriumchlorid | – | – | 59,5% |

Der Wirkstoff wird mit den Zusatzstoffen gut vermischt und in einer geeigneten Mühle gut vermahlen. Man erhält so Spritzpulver, die sich mit Wasser zu Suspensionen jeder gewünschten Konzentration verdünnen lassen.

| b) Emulsion-Konzentrate | a) | b) |
|---|---|---|
| Wirkstoff | 10% | 1% |
| Octylphenolpolyäthylenglykoläther (4–5 Mol AeO) | 3% | 3% |
| Ca-Dodecylbenzolsulfonat | 3% | 3% |
| Ricinusölpolyglykoläther (36 Mol AeO) | 4% | 4% |
| Cyclohexanon | 30% | 10% |
| Xylolgemisch | 50% | 79% |

Aus diesem Konzentrat können durch Verdünnen mit Wasser Emulsionen jeder gewünschten Konzentration hergestellt werden.

| c) Stäubemittel | a) | b) |
|---|---|---|
| Wirkstoff | 0,1% | 1% |
| Talkum | 99,9% | – |
| Kaolin | – | 99% |

Man erhält anwendungsfertige Stäubemittel, indem der Wirkstoff mit dem Träger vermischt und auf einer geeigneten Mühle vermahlen wird.

| d) Extruder Granulat | a) | b) |
|---|---|---|
| Wirkstoff | 10% | 1% |
| Na-Ligninsulfonat | 2% | 2% |
| Carboxymethylcellulose | 1% | 1% |
| Kaolin | 87% | 96% |

Der Wirkstoff wird mit den Zusatzstoffen vermischt, vermahlen und mit Wasser angefeuchtet. Dieses Gemisch wird extrudiert und anschließend im Luftstrom getrocknet.

| e) Umhüllungs-Granulat | |
|---|---|
| Wirkstoff | 3% |
| Polyäthylenglykol (MG 200) | 3% |
| Kaolin | 94% |

Der fein gemahlene Wirkstoff wird in einem Mischer auf das mit Polyäthylenglykol angefeuchtete Kaolin gleichmäßig aufgetragen. Auf diese Weise erhält man staubfreie Umhüllungs-Granulate.

| f) Suspensions-Konzentrat | a) | b) |
|---|---|---|
| Wirkstoff | 40% | 5% |
| Äthylenglykol | 10% | 10% |
| Nonylphenolpolyäthylenglykoläther (15 Mol AeO) | 6% | 1% |
| Na-Ligninsulfonat | 10% | 5% |
| Carboxymethylcellulose | 1% | 1% |
| 37%ige wäßrige Formaldehydlösung | 0,2% | 0,2% |
| Silikonöl in Form einer 75%igen wäßrigen Emulsion | 0,8% | 0,8% |
| Wasser | 32% | 77% |

Der fein gemahlene Wirkstoff wird mit den Zusatzstoffen innig vermischt. Man erhält so ein Suspension-Konzentrat, aus welchem durch Verdünnen mit Wasser Suspensionen jeder gewünschten Konzentration hergestellt werden können.

| g) Salzlösung | |
|---|---|
| Wirkstoff | 5% |
| Isopropylamin | 1% |
| Octylphenolpolyäthylenglykoläther (78 Mol AeO) | 3% |
| Wasser | 91% |

Biologische Beispiele

Beispiel 19:

Herbizidwirkung vor dem Auflaufen der Pflanzen

Im Gewächshaus werden Pflanzensamen in Blumentöpfe von 12–15 cm Durchmesser gesät. Unmittelbar danach wird die Erdoberfläche mit einer wäßrigen Dispersion oder Lösung der Wirkstoffe behandelt. Es werden Konzentrationen von 500 und 250 g Wirkstoffmenge pro Hektar angewendet. Die Töpfe werden dann im Gewächshaus bei einer Temperatur von 22–25 °C und 50–70% relativer Luftfeuchtigkeit gehalten. Nach 3 Wochen wird der Versuch ausgewertet.

Der Zustand der Pflanzen wird gemäß folgender Skala bewertet:

9 Pflanze gedeiht wie unbehandelte Kontrollpflanze, kein Schaden
8 sehr leichte phytotoxische Symptome
7 leichter Schaden
6 regenerierbarer Schaden
5 permanenter Schaden
4 Pflanze verkümmert
3 schwerer Schaden
2 sehr schwerer Schaden
1 Pflanze abgestorben oder hat nicht gekeimt.

Eine selektive Herbizidwirkung ist vorhanden, wenn bei derselben Aufwandmenge die Kulturpflanze eine Note 6–9 und die Unkräuter Noten 1–4 zeigen.

Die Resultate sind untenstehend zusammengefaßt.

| Verbindung No. | 1 | | 4 | | 6 | | 10 | | 13 | | 37 | |
|---|---|---|---|---|---|---|---|---|---|---|---|---|
| Aufwandmenge g/ha | 500 | 250 | 500 | 250 | 500 | 250 | 500 | 250 | 500 | 350 | 500 | 350 |
| Pflanze: | | | | | | | | | | | | |
| Weizen | 9 | 9 | 7 | 8 | 8 | 8 | 7 | 8 | 6 | 8 | 8 | 8 |
| Alopecurus myosuroides | 2 | 3 | 2 | 2 | 3 | 4 | 1 | 3 | 1 | 1 | 2 | 4 |
| Echinochloa crus galli | 3 | 4 | 4 | 4 | 4 | 4 | 3 | 4 | 1 | 2 | 2 | 2 |
| Cyperus esculentus | 3 | 4 | 3 | 4 | 3 | 4 | 2 | 3 | 1 | 1 | 1 | 1 |
| Abutilon sp. | 1 | 2 | 2 | 2 | 3 | 3 | 1 | 2 | 1 | 1 | 2 | 3 |
| Xanthium spinosum | 2 | 3 | 1 | 3 | 3 | 4 | 3 | 4 | 2 | 2 | 2 | 3 |
| Chenopodium album | 2 | 2 | 1 | 1 | 2 | 2 | 2 | 2 | 2 | 2 | 2 | 2 |
| Ipomoea purpurea | 2 | 2 | 2 | 3 | 2 | 2 | 2 | 2 | 2 | 2 | 2 | 2 |
| Sinapis alba | 1 | 1 | 2 | 2 | 2 | 2 | 2 | 2 | 2 | 2 | 2 | 2 |
| Galium aparine | 4 | 4 | 2 | 2 | 1 | 2 | 2 | 3 | 3 | 4 | 2 | 3 |
| Viola tricolor | 3 | 3 | 2 | 2 | 1 | 1 | 2 | 2 | 1 | 1 | 2 | 2 |

Beispiel 20:

Herbizidwirkung nach dem Auflaufen der Pflanzen (Kontaktwirkung)

Eine Anzahl Unkräuter und Kulturpflanzen, sowohl monokotyle wie dikotyle, wurden nach dem Auflaufen, im 4- bis 6-Blattstadium mit einer wäßrigen Wirkstoffdispersion von 4 kg AS/ha gespritzt und dann bei 24 bis 26 °C und 45–60% relativer Luftfeuchtigkeit gehalten. 15 Tage nach der Behandlung wird der Versuch ausgewertet und der Zustand der Pflanzen nach der oben gegebenen Skala bonitiert.

Die Resultate sind untenstehend zusammengefaßt.

| Verbindung No. | 1 | | 3 | | 4 | | 6 | | 7 | | 8 | |
|---|---|---|---|---|---|---|---|---|---|---|---|---|
| Aufwandmenge g/ha | 250 | 125 | 250 | 125 | 250 | 125 | 250 | 125 | 250 | 125 | 250 | 125 |
| Pflanze: | | | | | | | | | | | | |
| Weizen | 9 | 9 | 9 | 9 | 8 | 9 | 9 | 9 | 7 | 8 | 8 | 9 |
| Mais | 5 | 7 | 8 | 8 | 7 | 9 | 5 | 7 | 2 | 3 | 5 | 7 |
| Alopecurus myosuroides | 4 | 4 | 5 | 7 | 3 | 4 | 4 | 6 | 2 | 3 | 4 | 6 |
| Echinochloa crus galli | 8 | 8 | 8 | 9 | 8 | 9 | 7 | 8 | 4 | 4 | 2 | 6 |
| Cyperus esculentus | 4 | 4 | 3 | 4 | 3 | 3 | 3 | 4 | 3 | 3 | 4 | 6 |
| Abutilon sp. | 2 | 2 | 5 | 5 | 3 | 3 | 3 | 4 | 2 | 3 | 2 | 3 |
| Xanthium spinosum | 1 | 1 | 2 | 2 | 2 | 2 | 2 | 3 | 2 | 2 | 1 | 4 |
| Chenopodium album | 2 | 2 | 3 | 3 | 2 | 2 | 2 | 3 | 2 | 2 | 2 | 3 |
| Ipomoea purpurea | 4 | 4 | 3 | 4 | 3 | 4 | 3 | 4 | 3 | 4 | 3 | 5 |
| Sinapis alba | 2 | 3 | 2 | 3 | 2 | 2 | 2 | 2 | 2 | 2 | 2 | 3 |
| Galium aparine | 2 | 2 | 3 | 3 | 2 | 2 | 2 | 2 | 2 | 2 | 2 | 3 |
| Viola tricolor | 2 | 4 | 3 | 3 | 2 | 2 | 2 | 2 | 2 | 2 | 3 | 4 |

| Verbindung No. | 10 | | 19 | | 41 | | 73 | |
|---|---|---|---|---|---|---|---|---|
| Aufwandmenge g/ha | 250 | 125 | 250 | 125 | 250 | 125 | 250 | 125 |
| Pflanze: | | | | | | | | |
| Weizen | 9 | 9 | 3 | 4 | 5 | 7 | 6 | 8 |
| Mais | 5 | 7 | 3 | 8 | 4 | 8 | 4 | 8 |
| Alopecurus myosuroides | 2 | 3 | 3 | 4 | 2 | 2 | 3 | 5 |
| Echinochloa crus galli | 4 | 6 | 2 | 3 | 4 | 5 | 3 | 4 |
| Cyperus esculentus | 3 | 5 | 3 | 4 | 3 | 4 | 4 | 5 |
| Abutilon sp. | 3 | 3 | 3 | 4 | 2 | 2 | 2 | 3 |
| Xanthium spinosa | 2 | 4 | 3 | 3 | 1 | 1 | 2 | 2 |
| Chenopodium album | 2 | 3 | 3 | 4 | 2 | 3 | 2 | 2 |
| Ipomoea purpurea | 4 | 4 | 4 | 4 | 4 | 5 | 4 | 4 |
| Sinapis alba | 2 | 3 | 3 | 3 | 3 | 4 | 3 | 3 |
| Galium aparine | 3 | 4 | 4 | 4 | 3 | 4 | 3 | 5 |
| Viola tricolor | 3 | 4 | 4 | 5 | 2 | 3 | 2 | 3 |

Beispiel 21:
Keimhemmung an Lagerkartoffeln

Eine Anzahl im Handel erhältlicher Kartoffeln der Sorte «Urgenta» ohne Keime werden gewaschen und abgetrocknet. Danach werden die Kartoffeln für jeweils eine Minute in Wirkstoffemulsionen verschiedener Konzentration getaucht, in Kunststoffschalen auf Filterpapier ausgelegt und bei Temperaturen von 14 und 21 °C im Dunkeln bei 50% relativer Luftfeuchtigkeit gehalten. Die Auswertung erfolgte 34 Tage nach der Applikation. Gleichzeitig wird der Gewichtsverlust der Knollen und das Gewicht der Keime im Vergleich zur unbehandelten Kontrolle ermittelt. Die geprüften erfindungsgemäßen Verbindungen zeigen in diesem Versuch eine vollständige Verhinderung der Keimbildung. Gleichzeitig beträgt der Gewichtsverlust der Kartoffeln weniger als 10% des Gewichtsverlustes der Kontrollkartoffeln.

Beispiel 22:
Wuchshemmung bei tropischen Bodendecker-Leguminosen (cover crops)

Die Versuchspflanzen (centrosema plumieri und centrosema pubescens) werden bis zum ausgewachsenen Stadium herangezogen und bis auf eine Höhe von 60 cm zurückgeschnitten. Nach 7 Tagen wird der Wirkstoff als wäßrige Emulsion gespritzt. Die Versuchspflanzen werden bei 70% relativer Luftfeuchtigkeit und 6000 lux Kunstlicht, pro Tag 14 Stunden, bei Temperaturen von 27 bei Tag und 21 °C bei Nacht gehalten. 4 Wochen nach der Applikation wird der Versuch ausgewertet. Es werden dabei der Neuzuwachs im Vergleich zur Kontrolle abgeschätzt und gewogen und die Phytotoxizität bewertet. In diesem Versuch zeigen die mit den Wirkstoffen der allgemeinen Formel I behandelten Pflanzen eine deutliche Reduktion des Neuzuwachses (weniger als 20% des Neuzuwachses bei unbehandelten Kontrollpflanzen), ohne daß dabei die Versuchspflanzen geschädigt wurden.

## Patentansprüche

1. N-(Cyclopropyl-triazinyl- und -pyrimidinyl)-N'-(arylsulfonyl)-harnstoffe der allgemeinen Formel I

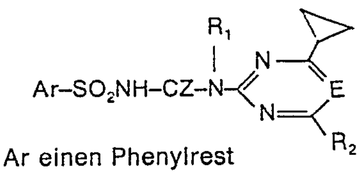

(I)

worin Ar einen Phenylrest

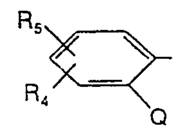

oder einen Naphthylrest

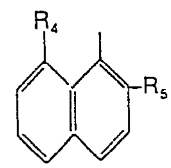

und

Q einen Rest X–A oder $R_3$,

A einen $C_3$–$C_6$-Alkinylrest, einen gegebenenfalls durch Halogen, $C_1$–$C_4$-Alkoxy, $C_1$–$C_4$-Alkylthio, $C_1$–$C_4$-Alkylsulfinyl, $C_1$–$C_4$-Alkylsulfonyl, $C_1$–$C_4$-Halogenalkoxy, $C_1$–$C_4$-Halogenalkylthio, $C_1$–$C_4$-Halogenalkylsulfinyl oder $C_1$–$C_4$-Halogenalkylsulfonyl substituierten $C_1$–$C_6$-Alkylrest oder einen gegebenenfalls durch die aufgezählten Reste substituierten $C_2$–$C_6$-Alkenylrest, einen Phenyl- oder Benzylrest,

E die Methingruppe oder Stickstoff,

X Sauerstoff, Schwefel, eine Sulfinyl- oder Sulfonylbrücke,

Z Sauerstoff oder Schwefel,

$R_1$ Wasserstoff, $C_1$–$C_4$-Alkyl oder $C_1$–$C_4$-Alkoxy,

$R_2$ Halogen, $C_1$–$C_3$-Alkyl, $C_1$–$C_3$-Halogenalkyl, $C_1$–$C_3$-Alkoxy, $C_1$–$C_3$-Halogenalkoxy, Amino, $C_1$–$C_3$-Alkylamino, Di-($C_1$–$C_3$-Alkyl)amino, $C_3$–$C_6$-Cycloalkyl oder $C_2$–$C_6$-Alkoxyalkyl,

$R_3$ Wasserstoff, Halogen, $C_1$–$C_5$-Alkyl, $C_2$–$C_5$-Alkenyl, $C_1$–$C_4$-Halogenalkyl, oder einen Rest $-X-R_6$, $-COZR_{11}$, $-NO_2$ oder $-CO-NR_8R_9$, $-CN$, $-COR_{10}$, $-NR_1R_7$, oder $-NR_1-COR_{12}$,

$R_4$ Wasserstoff, $C_1$–$C_4$-Alkyl, $C_1$–$C_4$-Alkoxy, $C_1$–$C_4$-Alkylthio, $C_1$–$C_4$-Halogenalkyl, $C_1$–$C_4$-Halogenalkoxy, Halogen oder Alkoxyalkyl mit höchstens 4 Kohlenstoffatomen,

$R_5$ dasselbe wie $R_3$ aber unabhängig davon,

$R_6$ und $R_7$ je $C_1$–$C_5$-Alkyl, $C_2$–$C_5$-Alkenyl oder $C_2$–$C_6$-Alkinyl,

$R_8$ und $R_9$ unabhängig voneinander Wasserstoff, $C_1$–$C_5$-Alkyl, $C_2$–$C_5$-Alkenyl oder $C_2$–$C_6$-Alkinyl,

$R_{10}$ Wasserstoff, $C_1$–$C_4$-Aklyl oder $C_1$–$C_3$-Halogenalkyl,

$R_{11}$ Wasserstoff, $C_1$–$C_4$-Alkyl, $C_1$–$C_3$-Halogenalkyl, $C_3$–$C_5$-Alkenyl, $C_3$–$C_5$-Alkinyl, Phenyl oder Benzyl und

$R_{12}$ dasselbe wie $R_1$ aber unabhängig davon bedeuten und deren Salze.

2. Die Verbindungen der allgemeinen Formel I gemäß Anspruch 1, in denen Z Sauerstoff bedeutet.

3. Die Verbindungen der allgemeinen Formel I gemäß Anspruch 1, in denen Ar einen in ortho-Stellung durch Q substituierten Phenylrest, $R_1$ Wasserstoff oder Methyl, $R_2$ einen $C_1$–$C_3$-Alkyl-, $C_1$–$C_3$-Haloalkyl-, $C_1$–$C_3$-Alkoxy-, $C_1$–$C_3$-Haloalkoxy- oder $C_1$–$C_3$-Alkylaminorest, E das Stickstoffatom oder die Methingruppe und Z Sauerstoff bedeuten.

4. Die Verbindungen der allgemeinen Formel I gemäß Anspruch 1, worin Ar einen in ortho-Stellung durch $C_1$–$C_4$-Alkoxycarbonyl substituierten Phenylrest, E das Stickstoffatom oder die Methingruppe, $R_1$ Wasserstoff oder Methyl, $R_2$ einen $C_1$–$C_3$-Alkyl-, $C_1$–$C_3$-Haloalkyl-, $C_1$–$C_3$-Alkoxy-, $C_1$–$C_3$-Haloalkoxy- oder $C_1$–$C_3$-Alkylaminorest und Z Sauerstoff bedeuten.

5. Die Verbindungen der allgemeinen Formel I gemäß Anspruch 1, worin Ar einen in ortho-Stellung durch $C_1$–$C_3$-Haloalkoxy substituierten Phenylrest, E das Stickstoffatom oder die Methingruppe, $R_1$ Wasserstoff oder Methyl, $R_2$ einen $C_1$–$C_3$-Alkyl-, $C_1$–$C_3$-Haloalkyl-, $C_1$–$C_3$-Alkoxy-, $C_1$–$C_3$-Haloalkoxy- oder $C_1$–$C_3$-Alkylaminorest und Z Sauerstoff bedeuten.

6. Die Verbindungen der allgemeinen Formel I gemäß Anspruch 1, worin Ar einen in ortho-Stellung durch Halogen- oder Nitro substituierten Phenylrest, E das Stickstoffatom oder die Methingruppe, $R_1$ Wasserstoff oder Methyl, $R_2$ einen $C_1$–$C_3$-Alkyl-, $C_1$–$C_3$-Haloalkyl, $C_1$–$C_3$-Alkoxy-, $C_1$–$C_3$-Haloalkoxy- oder $C_1$–$C_3$-Alkylaminorest und Z Sauerstoff bedeuten.

7. N-(4-Cyclopropyl-6-methylpyrimidin-2-yl)-N'-(2-methoxycarbonylbenzosulfonyl)-harnstoff
gemäß Anspruch 1.

8. N-(4-Cyclopropyl-6-methoxy-1,3,5-triazin-2-yl)-N'-(2-methoxycarbonylbenzolsulfonyl)-harnstoff
gemäß Anspruch 1.

9. N-(4-Cyclopropyl-6-äthoxy-1,3,5-triazin-2-yl)-N'-(2-difluormethoxybenzolsulfonyl)-harnstoff
gemäß Anspruch 1.

10. Verfahren zur Herstellung der Harnstoffe der allgemeinen Formel I gemäß Anspruch 1, dadurch gekennzeichnet, daß man ein Arylsulfonamid der Formel II

$$Ar–SO_2NH_2 \quad (II),$$

worin Ar die unter allgemeiner Formel I im Anspruch 1 gegebene Bedeutung hat, in Gegenwart einer Base mit einem N-Pyrimidinyl- oder -triazinylcarbamat der Formel III

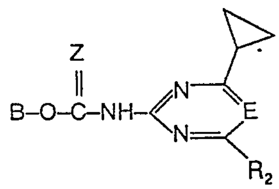
(III),

worin E, $R_2$ und Z die unter allgemeiner Formel I gegebene Bedeutung haben, und B–O einen abspaltbaren Phenoxy-, Phenylalkoxy- oder Alkoxyrest bedeutet, umsetzt und gegebenenfalls in ihre Salze überführt.

11. Verfahren zur Herstellung der Harnstoffe der allgemeinen Formel I gemäß Anspruch 1, dadurch gekennzeichnet, daß man ein Arylsulfonylisocyanat oder -isothiocyanat der Formel IV

$$Ar–SO_2–N=C=Z \quad (IV),$$

worin Ar und Z die unter allgemeiner Formel I gegebene Bedeutung haben, gegebenenfalls in Gegenwart einer Base, mit einem Amin der Formel V

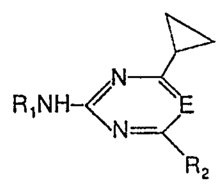
(V),

worin E, $R_1$ und $R_2$ die unter allgemeiner Formel I gegebene Bedeutung haben, umsetzt und gegebenenfalls in ihre Salze überführt.

12. Verfahren zur Herstellung der Harnstoffe der allgemeinen Formel I gemäß Anspruch 1, dadurch gekennzeichnet, daß man Sulfonamide der Formel II des Anspruchs 10 gegebenenfalls in Gegenwart einer Base mit einem Isocyanat oder Isothiocyanat der Formel VI

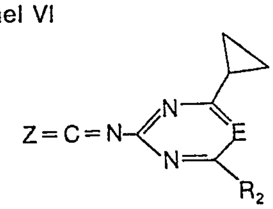
(VI),

worin E, $R_2$ und Z die unter allgemeiner Formel I gegebene Bedeutungen haben, umsetzt und gegebenenfalls in ihre Salze überführt.

13. Verfahren zur Herstellung der Harnstoffe der allgemeinen Formel I gemäß Anspruch 1, dadurch gekennzeichnet, daß man ein N-Phenylsulfonylcarbamat der Formel VII

$$Ar–SO_2NHCOO–B \quad (VII),$$

worin Ar die unter allgemeiner Formel I gegebene Bedeutung hat und B–O– einen abspaltbaren Phenoxy-, Phenylalkoxy- oder Alkoxyrest bedeutet, mit einem Amin der im Anspruch 11 gegebenen Formel V umsetzt und gegebenenfalls in ihre Salze überführt.

14. Verfahren zur Herstellung von Additionssalzen der allgemeinen Formel I gemäß einem der Ansprüche 10 bis 13, dadurch gekennzeichnet, daß man einen Sulfonylharnstoff der allgemeinen Formel I mit einem Amin, einem Alkalimetall- oder Erdalkalimetallhydroxid oder einer quaternären Ammoniumbase umsetzt.

15. Ein herbizides und den Pflanzenwuchs hemmendes Mittel, dadurch gekennzeichnet, daß es neben Träger- und/oder anderen Zuschlagstoffen als Wirkstoff mindestens einen N-(Cyclopropyl-triazinyl- oder -pyrimidinyl)-N'-(arylsulfonyl)-harnstoff der allgemeinen Formel I gemäß Anspruch 1 enthält.

16. Die Verwendung der N-(Cyclopropyl-triazinyl- und -pyrimidinyl)-N'-(arylsulfonyl)-harnstoffe der allgemeinen Formel I gemäß Anspruch 1, oder sie enthaltender Mittel zur Bekämpfung unerwünschten Pflanzenwachstums.

17. Die Verwendung der N-(Cyclopropyl-triazinyl- und -pyrimidinyl)-N'-(arylsulfonyl)-harnstoffe der allgemeinen Formel I gemäß Anspruch 1, oder sie enthaltender Mittel zur Hemmung des Pflanzenwachstums.

18. Die Verwendung der N-(Cyclopropyl-triazinyl- und -pyrimidinyl)-N'-(arylsulfonyl)-harnstoffe der allgemeinen Formel I gemäß Anspruch 1, oder sie enthaltender Mittel zur selektiven pre- oder postemergenten Bekämpfung von Unkräutern in Nutzpflanzenkulturen.

19. Die Verwendung der N-(Cyclopropyl-triazinyl- und -pyrimidinyl)-N'-(arylsulfonyl)-harnstoffe der allgemeinen Formel I gemäß Anspruch 1 oder sie enthaltender Mittel zur Unterdrückung des Pflanzenwachstums über das 2-Blattstadium hinaus, dadurch gekennzeichnet, daß die Wirkstoffe preemergent angewendet werden.

20. 2-Amino-4-cyclopropyl-pyrimidine der Formel V

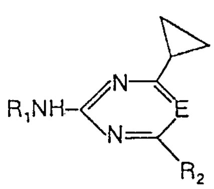

(V),

worin E, $R_1$ und $R_2$ die unter allgemeiner Formel I gegebene Bedeutung haben, mit der Maßnahme, daß $R_2$ nicht Cl, OH oder $OCH_3$ bedeuten kann, als Zwischenprodukte zur Herstellung der Harnstoffe der allgemeinen Formel I.

21. 4-Cyclopropyl-1,3,5-triazine der Formel VIII

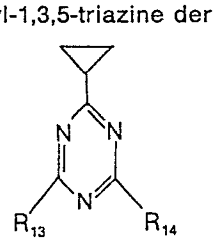

worin $R_{13}$ Trichlormethyl oder $-NH-R_1$ bedeutet und $R_{14}$ Halogen, $C_{1-3}$ Halogenalkyl, $C_{1-4}$ Allyl oder $C_1$–$C_4$-Alkoxy bedeutet, als Zwischenprodukte zur Herstellung der Harnstoffe der allgemeinen Formel I.

22. 2-Amino-4-cyclopropyl-6-methoxy-1,3,5-triazin
gemäß Anspruch 21.

23. 2-Amino-4-cyclopropyl-6-äthoxy-1,3,5-triazin
gemäß Anspruch 21.

24. 2-Amino-4-cyclopropyl-6-trichlormethyl-1,3,5-triazin
gemäß Anspruch 21.

25. 2-Cyclopropyl-4,6-bis-(trichlormethyl)-1,3,5-triazin
gemäß Anspruch 21.

26. N-(4-Cyclopropyl-6-methylthio-1,3,5-triazin-2-yl)-N'-(chlorphenylsulfonyl)-harnstoff,
N-(4-Cyclopropyl-6-methylthiopyrimidin-2-yl)-N'-(2-methoxycarbonylphenylsulfonyl)-harnstoff,
N-(4-Cyclopropyl-6-methylthiopyrimidin-2-yl)-N'-(2-nitrophenylsulfonyl)-harnstoff,
N-(4-Cyclopropyl-6-methylthiopyrimidin-2-yl)-N'-[2-(2-chlorethoxy)-phenylsulfonyl]-harnstoff oder
N-(4-Cyclopropyl-6-methylthiopyrimidin-2-yl)-N'-[2-(1,2-dichlorethenyloxy)-phenylsulfonyl]-harnstoff.

27. Ein herbizides oder den Pflanzenwuchs hemmendes Mittel, dadurch gekennzeichnet, daß es neben Träger- und/oder anderen Zuschlagstoffen als Wirkstoff mindestens einen N-(Cyclopropyl-triazinyl- oder -pyrimidinyl)-N'-(arylsulfonyl)-Harnstoff gemäß Anspruch 26 enthält.

28. Verfahren zur Herstellung von
N-(4-Cyclopropyl-6-methylthio-1,3,5-triazin-2-yl)-N'-(chlorphenylsulfonyl)-harnstoff,
N-(4-Cyclopropyl-6-methylthiopyrimidin-2-yl)-N'-(2-methoxycarbonylphenylsulfonyl)-harnstoff,
N-(4-Cyclopropyl-6-methylthiopyrimidin-2-yl)-N'-(2-nitrophenylsulfonyl)-harnstoff,
N-(4-Cyclopropyl-6-methylthiopyrimidin-2-yl)-N'-[2-(2-chlorethoxy)-phenylsulfonyl]-harnstoff oder
N-(4-Cyclopropyl-6-methylthiopyrimidin-2-yl)-N'-[2-(1,2-dichlorethenyloxy)-phenylsulfonyl]-harnstoff
dadurch gekennzeichnet, daß man ein Phenylsulfonylisocyanat der Formel IVa

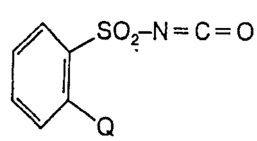

(IVa),

worin Q Chlor, Methoxycarbonyl, Nitro, 2-Chlorethoxy oder 1,2-Dichlorethenyloxy bedeutet, mit einer Verbindung der Formel Va

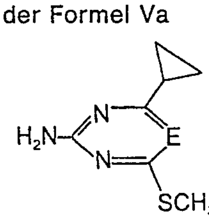

(Va),

worin E N oder CH bedeutet, umsetzt.

29. Die Verwendung von
N-(4-Cyclopropyl-6-methylthio-1,3,5-triazin-2-yl)-N'-(chlorphenylsulfonyl)-harnstoff,
N-(4-Cyclopropyl-6-methylthiopyrimidin-2-yl)-N'-(2-methoxycarbonylphenylsulfonyl)-harnstoff,
N-(4-Cyclopropyl-6-methylthiopyrimidin-2-yl)-N'-(2-nitrophenylsulfonyl)-harnstoff,
N-(4-Cyclopropyl-6-methylthiopyrimidin-2-yl)-N'-[2-(2-chlorethoxy)-phenylsulfonyl]-harnstoff oder
N-(4-Cyclopropyl-6-methylthiopyrimidin-2-yl)-N'-[2-(1,2-dichlorethenyloxy)-phenylsulfonyl]-harnstoff
oder sie enthaltender Mittel zur Bekämpfung unerwünschten Pflanzenwachstums.

## Claims

1. N-(Cyclopropyl-triazinyl- and -pyrimidinyl)-N'-(arylsulfonyl)-ureas of the general formula I

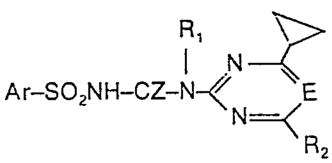
(I)

wherein Ar is a phenyl group

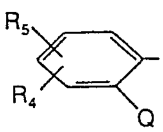

or a naphthyl group

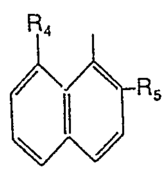

and

Q is a group X–A or $R_3$,

A is a $C_3$–$C_6$alkynyl group, a $C_1$–$C_6$alkyl group which is unsubstituted or substituted by halogen, $C_1$–$C_4$alkoxy, $C_1$–$C_4$alkylthio, $C_1$–$C_4$alkylsulfinyl, $C_1$–$C_4$alkylsulfonyl, $C_1$–$C_4$haloalkoxy, $C_1$–$C_4$haloalkylthio, $C_1$–$C_4$haloalkylsulfinyl or by $C_1$–$C_4$haloalkylsulfonyl, or a $C_2$–$C_6$alkenyl group which is unsubstituted or substituted by the groups mentioned, or is a phenyl or benzyl group,

E is the methine group or nitrogen,

X is oxygen, sulfur, or a sulfinyl or sulfonyl bridge,

Z is oxygen or sulfur,

$R_1$ is hydrogen, $C_1$–$C_4$alkyl or $C_1$–$C_4$alkoxy,

$R_2$ is halogen, $C_1$–$C_3$alkyl, $C_1$–$C_3$haloalkyl, $C_1$–$C_3$alkoxy, $C_1$–$C_3$haloalkoxy, amino, $C_1$–$C_3$alkylamino, di-($C_1$–$C_3$alkyl)amino, $C_3$–$C_6$cycloalkyl or $C_2$–$C_6$alkoxyalkyl,

$R_3$ is hydrogen, halogen, $C_1$–$C_5$alkyl, $C_2$–$C_5$alkenyl, $C_1$–$C_4$haloalkyl, or a group $-X-R_6$, $-COZR_{11}$, $-NO_2$ or $-CO-NR_8R_9$, –CN, $-COR_{10}$, $-NR_1R_7$ or $-NR_1-COR_{12}$,

$R_4$ is hydrogen, $C_1$–$C_4$alkyl, $C_1$–$C_4$alkoxy, $C_1$–$C_4$alkylthio, $C_1$–$C_4$haloalkyl, $C_1$–$C_4$haloalkoxy, halogen, or alkoxyalkyl having at most 4 carbon atoms,

$R_5$ is the same as $R_3$ but independently thereof,

$R_6$ and $R_7$ are each $C_1$–$C_5$alkyl, $C_2$–$C_5$alkenyl or $C_2$–$C_6$alkynyl,

$R_8$ and $R_9$ independently of one another are each hydrogen, $C_1$–$C_5$alkyl, $C_2$–$C_5$alkenyl or $C_2$–$C_6$alkynyl,

$R_{10}$ is hydrogen, $C_1$–$C_4$alkyl or $C_1$–$C_3$haloalkyl,

$R_{11}$ is hydrogen, $C_1$–$C_4$alkyl, $C_1$–$C_3$haloalkyl, $C_3$–$C_5$alkenyl, $C_3$–$C_5$alkynyl, phenyl or benzyl, and

$R_{12}$ is the same as $R_1$ but independently thereof, and salts thereof.

2. The compounds of the general formula I according to claim 1, wherein Z is oxygen.

3. The compounds of the general formula I according to claim 1, wherein Ar is a phenyl group substituted in the ortho-position by Q, $R_1$ is hydrogen or methyl, $R_2$ is a $C_1$–$C_3$alkyl, $C_1$–$C_3$haloalkyl, $C_1$–$C_3$alkoxy, $C_1$–$C_3$haloalkoxy or $C_1$–$C_3$alkylamino group, E is the nitrogen atom or the methine group, and Z is oxygen.

4. The compounds of the general formula I according to claim 1, wherein Ar is a phenyl group substituted in the ortho-position by $C_1$–$C_4$alkoxycarbonyl, E is the nitrogen atom or the methine group, $R_1$ is hydrogen or methyl, $R_2$ is a $C_1$–$C_3$alkyl, $C_1$–$C_3$haloalkyl, $C_1$–$C_3$alkoxy, $C_1$–$C_3$haloalkoxy or $C_1$–$C_3$alkylamino group, and Z is oxygen.

5. The compounds of the general formula I according to claim 1, wherein Ar is a phenyl group substituted in the ortho-position by $C_1$–$C_3$haloalkoxy, E is the nitrogen atom or the methine group, $R_1$ is hydrogen or methyl, $R_2$ is a $C_1$–$C_3$alkyl, $C_1$–$C_3$haloalkyl, $C_1$–$C_3$alkoxy, $C_1$–$C_3$haloalkoxy or $C_1$–$C_3$alkylamino group, and Z is oxygen.

6. The compounds of the general formula I according to claim 1, wherein Ar is a phenyl group substituted in the ortho-position by halogen or by nitro, E is the nitrogen atom or the methine group, $R_1$ is hydrogen or methyl, $R_2$ is a $C_1$–$C_3$alkyl, $C_1$–$C_3$haloalkyl, $C_1$–$C_3$alkoxy, $C_1$–$C_3$haloalkoxy or $C_1$–$C_3$alkylamino group, and Z is oxygen.

7. N-(4-Cyclopropyl-6-methylpyrimidin-2-yl)-N'-(2-methoxycarbonylbenzenesulfonyl)-urea
according to claim 1.

8. N-(4-Cyclopropyl-6-methoxy-1,3,5-triazin-2-yl)-N'-(2-methoxycarbonylbenzenesulfonyl)-urea
according to claim 1.

9. N-(4-Cyclopropyl-6-ethoxy-1,3,5-triazin-2-yl)-N'-(2-difluoromethoxybenzenesulfonyl)-urea
according to claim 1.

10. A process for producing the ureas of the general formula I according to claim 1, which process comprises reacting an arylsulfonamide of the formula II

$$Ar-SO_2NH_2 \qquad (II),$$

wherein Ar has the meaning given under the general formula I in claim 1, in the presence of a base,

with an N-pyrimidinyl- or -triazinylcarbamate of the formula III

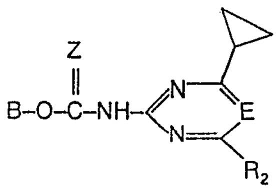

(III),

wherein E, $R_2$ and Z have the meanings given under the general formula I, and B–O is a phenoxy, phenylalkoxy or alkoxy group which can be detached, and optionally converting the compounds obtained into their salts.

11. A process for producing the ureas of the general formula I according to claim 1, which process comprises reacting an arylsulfonylisocyanate or -isothiocyanate of the formula IV

$$Ar{-}SO_2{-}N=C=Z \qquad (IV),$$

wherein Ar and Z have the meanings given under the general formula I, in the presence or absence of a base, with an amine of the formula V

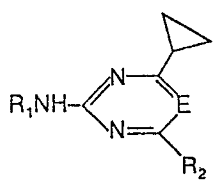

(V),

wherein E, $R_1$ and $R_2$ have the meanings given under the general formula I, and optionally converting the compounds obtained into their salts.

12. A process for producing the ureas of the general formula I according to claim 1, which process comprises reacting sulfonamides of the formula II in claim 10, in the presence or absence of a base, with an isocyanate or isothiocyanate of the formula VI

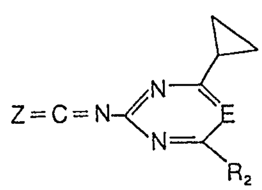

(VI),

wherein E, $R_2$ and Z have the meanings given under the general formula I, and optionally converting the compounds obtained into their salts.

13. A process for producing the ureas of the general formula I according to claim 1, which process comprises reacting an N-phenylsulfonylcarbamate of the formula VII

$$Ar{-}SO_2NHCOO{-}B \qquad (VII),$$

wherein Ar has the meaning given under the general formula I, and B–O– is a phenoxy, phenylalkoxy or alkoxy group which can be detached, with an amine of the formula V given in claim 11, and optionally converting the compounds obtained into their salts.

14. A process for producing addition salts of the general formula I according to any one of claims 10 to 13, which process comprises reacting a sulfonylurea of the general formula I with an amine, an alkali metal hydroxide or alkaline earth metal hydroxide or with a quaternary ammonium base.

15. A herbicidal and plant-growth-reducing composition which contains, as active ingredient, at least one N-(cyclopropyl-triazinyl- or -pyrimidinyl)-N′-(arylsulfonyl)-urea of the general formula I according to claim 1, together with carriers and/or other additives.

16. The use of N-(cyclopropyl-triazinyl- and -pyrimidinyl)-N′-(arylsulfonyl)-ureas of the general formula I according to claim 1, or of compositions containing them, for controlling undesirable plant growth.

17. The use of N-(cyclopropyl-triazinyl- and -pyrimidinyl)-N′-(arylsulfonyl)-ureas of the general formula I according to claim 1, or of compositions containing them, for reducing plant growth.

18. The use of N-(cyclopropyl-triazinyl- and -pyrimidinyl)-N′-(arylsulfonyl)-ureas of the general formula I according to claim 1, or of compositions containing them, for the selective pre- or post-emergence controlling of weeds in crops of useful plants.

19. The use of N-(cyclopropyl-triazinyl- and -pyrimidinyl)-N′-(arylsulfonyl)-ureas of the general formula I according to claim 1, or of compositions containing them, for suppressing plant growth beyond the two-leaf stage, which comprises applying the active ingredients pre-emergence.

20. 2-Amino-4-cyclopropyl-pyrimidines of the formula V

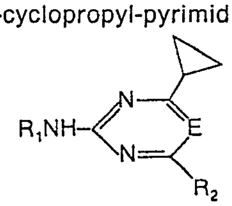

(V),

wherein E, $R_1$ and $R_2$ have the meanings given under the general formula I, with the proviso that $R_2$ cannot be Cl, OH or $OCH_3$, as intermediates for producing the ureas of the general formula I.

21. 4-Cyclopropyl-1,3,5-triazines of the formula VIII

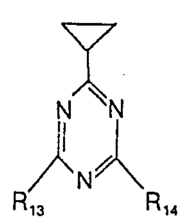

(VIII),

wherein $R_{13}$ is trichloromethyl or $-NH-R_1$, and $R_{14}$ is halogen, $C_{1-3}$haloalkyl, $C_{1-4}$allyl or $C_1$–$C_4$alkoxy, as intermediates for producing the ureas of the general formula I.

22. 2-Amino-4-cyclopropyl-6-methoxy-1,3,5-triazine

according to claim 21.

23. 2-Amino-4-cyclopropyl-6-ethoxy-1,3,5-triazine

according to claim 21.

24. 2-Amino-4-cyclopropyl-6-trichloromethyl-1,3,5-triazine
according to claim 21.

25. 2-Cyclopropyl-4,6-bis(trichloromethyl)-1,3,5-triazine
according to claim 21.

26. N-(4-Cyclopropyl-6-methylthio-1,3,5-triazin-2-yl)-N'-(chlorophenylsulfonyl)-urea,
N-(4-cyclopropyl-6-methylthiopyrimidin-2-yl)-N'-(2-methoxycarbonylphenylsulfonyl)-urea,
N-(4-cyclopropyl-6-methylthiopyrimidin-2-yl)-N'-(2-nitrophenylsulfonyl)-urea,
N-(4-cyclopropyl-6-methylthiopyrimidin-2-yl)-N'-[2-(2-chloroethoxy)-phenylsulfonyl]-urea
or
N-(4-cyclopropyl-6-methylthiopyrimidin-2-yl)-N'-[2-(1,2-dichloroethenyloxy)-phenylsulfonyl]-urea.

27. A herbicidal or plant-growth-reducing composition which contains, as active ingredient, at least one N-(cyclopropyl-triazinyl- or -pyrimidinyl)-N'-(arylsulfonyl)-urea according to claim 26, together with carriers and/or other additives.

28. A process for producing
N-(4-cyclopropyl-6-methylthio-1,3,5-triazin-2-yl)-N'-(chlorophenylsulfonyl)-urea,
N-(4-cyclopropyl-6-methylthiopyrimidin-2-yl)-N'-(2-methoxycarbonylphenylsulfonyl)-urea,
N-(4-cyclopropyl-6-methylthiopyrimidin-2-yl)-N'-(2-nitrophenylsulfonyl)-urea,
N-(4-cyclopropyl-6-methylthiopyrimidin-2-yl)-N'-[2-(2-chloroethoxy)-phenylsulfonyl]-urea
or
N-(4-cyclopropyl-6-methylthiopyrimidin-2-yl)-N'-[2-(1,2-dichloroethenyloxy)-phenylsulfonyl]-urea,
which process comprises reacting a phenylsulfonylisocyanate of the formula IVa

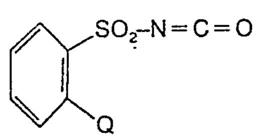
(IVa),

wherein Q is chlorine, methoxycarbonyl, nitro, 2-chloroethoxy or 1,2-dichloroethenyloxy, with a compound of the formula Va

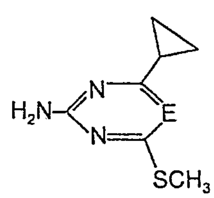
(Va),

wherein E is N or CH.

29. The use of N-(4-cyclopropyl-6-methylthio-1,3,5-triazin-2-yl)-N'-(chlorophenylsulfonyl)-urea,
N-(4-cyclopropyl-6-methylthiopyrimidin-2-yl)-N'-(2-methoxycarbonylphenylsulfonyl)-urea,
N-(4-cyclopropyl-6-methylthiopyrimidin-2-yl)-N'-(2-nitrophenylsulfonyl)-urea,
N-(4-cyclopropyl-6-methylthiopyrimidin-2-yl)-N'-[2-(2-chloroethoxy)-phenylsulfonyl]-urea
or
N-(4-cyclopropyl-6-methylthiopyrimidin-2-yl)-N'-[2-(1,2-dichloroethenyloxy)-phenylsulfonyl]-urea,
or of compositions containing them, for controlling undesirable plant growth.

## Revendications

1. N-(cyclopropyl-triazinyl- et -pyrimidinyl)-N'-(arylsulfonyl)-urées de formule générale I

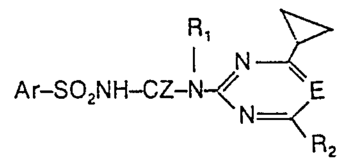
(I)

dans laquelle Ar représente un groupe phényle

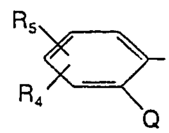

ou un groupe naphtyle

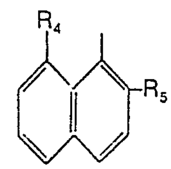

et

Q représente un groupe X–A ou $R_3$,

A représente un groupe alcynyle en $C_3$–$C_6$, un groupe alkyle en $C_1$–$C_6$ éventuellement substitué par des halogènes, des groupes alcoxy en $C_1$–$C_4$, alkylthio en $C_1$–$C_4$, alkylsulfinyle en $C_1$–$C_4$, alkylsulfonyle en $C_1$–$C_4$, halogénoalcoxy en $C_1$–$C_4$, halogénoalkylthio en $C_1$–$C_4$, halogénoalkylsulfinyle en $C_1$–$C_4$ ou halogénoalkylsulfonyle en $C_1$–$C_4$, ou un groupe alcényle en $C_2$–$C_6$ portant éventuellement les substituants qu'on vient d'énumérer, un groupe phényle ou benzyle,

E représente le groupe méthine ou l'azote,

X représente l'oxygène, le soufre, un pont sulfinyle ou sulfonyle,

Z représente l'oxygène ou le soufre,

$R_1$ représente l'hydrogène, un groupe alkyle en $C_1$–$C_4$ ou alcoxy en $C_1$–$C_4$,

$R_2$ représente un halogène, un groupe alkyle en $C_1$–$C_3$, halogénoalkyle en $C_1$–$C_3$, alcoxy en $C_1$–$C_3$, halogénoalcoxy en $C_1$–$C_3$, amino, alkylamino en $C_1$–$C_3$, di-(alkyle en $C_1$–$C_3$)-amino, cycloalkyle en $C_3$–$C_6$ ou alcoxyalkyle en $C_2$–$C_6$,

$R_3$ représente l'hydrogène, un halogène, un groupe alkyle en $C_1$–$C_5$, alcényle en $C_2$–$C_5$, halogénoalkyle en $C_1$–$C_4$ ou un groupe $-X-R_6$, $-COZR_{11}$, $-NO_2$ ou $-CO-NR_8R_9$, $-CN$, $-COR_{10}$, $-NR_1R_7$ ou $-NR_1-COR_{12}$,

$R_4$ représente l'hydrogène, un groupe alkyle en $C_1$–$C_4$, alcoxy en $C_1$–$C_4$, alkylthio en $C_1$–$C_4$, halogénoalkyle en $C_1$–$C_4$, halogénoalcoxy en $C_1$–$C_4$, un halogène ou un groupe alcoxyalkyle contenant au maximum 4 atomes de carbone,

$R_5$ a les mêmes significations que $R_3$ mais indépendamment de celui-ci,

$R_6$ et $R_7$ représentent chacun un groupe alkyle en $C_1$–$C_5$, alcényle en $C_2$–$C_5$, ou alcynyle en $C_2$–$C_6$,

$R_8$ et $R_9$ représentent chacun, indépendamment l'un de l'autre, l'hydrogène, un groupe alkyle en $C_1$–$C_5$, alcényle en $C_2$–$C_5$ ou alcynyle en $C_2$–$C_6$,

$R_{10}$ représente l'hydrogène, un groupe alkyle en $C_1$–$C_4$ ou halogénoalkyle en $C_1$–$C_3$,

$R_{11}$ représente l'hydrogène, un groupe alkyle en $C_1$–$C_4$, halogénoalkyle en $C_1$–$C_3$, alcényle en $C_3$–$C_5$, alcynyle en $C_3$–$C_5$, phényle ou benzyle, et

$R_{12}$ a les mêmes significations que $R_1$ mais indépendamment de celui-ci, et leurs sels.

2. Les composés de formule générale I selon la revendication 1, dans lesquels Z représente l'oxygène.

3. Les composés de formule générale I selon la revendication 1, dans lesquels Ar représente un groupe phényle substitué en position ortho par Q, $R_1$ représente l'hydrogène ou un groupe méthyle, $R_2$ représente un groupe alkyle en $C_1$–$C_3$, halogénoalkyle en $C_1$–$C_3$, alcoxy en $C_1$–$C_3$, halogénoalcoxy en $C_1$–$C_3$ ou alkylamino en $C_1$–$C_3$, E représente l'atome d'azote ou le groupe méthine et Z représente l'oxygène.

4. Les composés de formule générale I selon la revendication 1, dans lesquels Ar représente un groupe phényle substitué en position ortho par un groupe (alcoxy en $C_1$–$C_4$)-carbonyle, E représente l'atome d'azote ou le groupe méthine, $R_1$ représente l'hydrogène ou un groupe méthyle, $R_2$ représente un groupe alkyle en $C_1$–$C_3$, halogénoalkyle en $C_1$–$C_3$, alcoxy en $C_1$–$C_3$, halogénoalcoxy en $C_1$–$C_3$ ou alkylamino en $C_1$–$C_3$ et Z représente l'oxygène.

5. Les composés de formule générale I selon la revendication 1, dans lesquels Ar représente un groupe phényle substitué en position ortho par un groupe halogénoalcoxy en $C_1$–$C_3$, E représente l'atome d'azote ou le groupe méthine, $R_1$ représente l'hydrogène ou un groupe méthyle, $R_2$ représente un groupe alkyle en $C_1$–$C_3$, halogénoalkyle en $C_1$–$C_3$, alcoxy en $C_1$–$C_3$, halogénoalcoxy en $C_1$–$C_3$ ou alkylamino en $C_1$–$C_3$ et Z représente l'oxygène.

6. Les composés de formule générale I selon la revendication 1, dans lesquels Ar représente un groupe phényle substitué en position ortho par un halogène ou un groupe nitro, E représente l'atome d'azote ou le groupe méthine, $R_1$ représente l'hydrogène ou un groupe méthyle, $R_2$ représente un groupe alkyle en $C_1$–$C_3$, halogénoalkyle en $C_1$–$C_3$, alcoxy en $C_1$–$C_3$, halogénoalcoxy en $C_1$–$C_3$ ou alkylamino en $C_1$–$C_3$ et Z représente l'oxygène.

7. La N-(4-cyclopropyl-6-méthylpyrimidine-2-yl)-N'-(2-méthoxycarbonylbenzosulfonyl)-urée

selon la revendication 1.

8. La N-(4-cyclopropyl-6-méthoxy-1,3,5-triazine-2-yl)-N'-(2-méthoxycarbonylbenzène-sulfonyl)-urée

selon la revendication 1.

9. La N-(4-cyclopropyl-6-éthoxy-1,3,5-triazine-2-yl)-N'-(2-difluorométhoxybenzène-sulfonyl)-urée

selon la revendication 1.

10. Procédé de préparation des urées de formule générale I selon la revendication 1, caractérisé en ce que l'on fait réagir un arylsulfonamide de formule II

$$Ar{-}SO_2NH_2 \qquad (II)$$

dans laquelle Ar a les significations indiquées en référence à la formule générale I dans la revendication 1, en présence d'une base, avec un N-pyrimidinyl- ou -triazinyl-carbamate de formule III

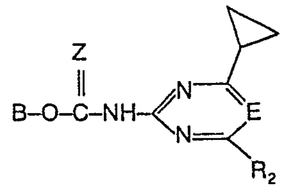

(III),

dans laquelle E, $R_2$ et Z ont les significations indiquées en référence à la formule générale I et B–O représente un groupe éliminable phénoxy, phénylalcoxy ou alcoxy, et le cas échéant, on convertit en les sels.

11. Procédé de préparation des urées de formule générale I selon la revendication 1, caractérisé en ce que l'on fait réagir un arylsulfonylisocyanate ou -isothiocyanate de formule IV

$$Ar{-}SO_2{-}N{=}C{=}Z \qquad (IV)$$

dans laquelle Ar et Z ont les significations indiquées en référence à la formule générale I, éventuellement en présence d'une base, avec une amine de formule V

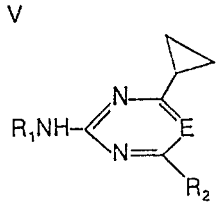

(V),

dans laquelle E, $R_1$ et $R_2$ ont les significations indiquées en référence à la formule générale I, et le cas échéant, on convertit en les sels.

12. Procédé de préparation des urées de formule générale I selon la revendication 1, caractérisé en ce que l'on fait réagir des sulfonamides de formule II de la revendication 10, éventuellement en présence d'une base, avec un isocyanate ou isothiocyanate de formule VI

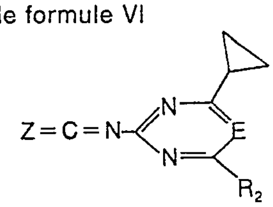

(VI),

dans laquelle E, $R_2$ et Z ont les significations indiquées en référence à la formule générale I, et le cas échéant, on convertit en les sels.

13. Procédé de préparation des urées de formule générale I selon la revendication 1, caractérisé en ce que l'on fait réagir un N-phénylsulfonylcarbamate de formule VII

$Ar–SO_2–NHCOO–B$ (VII)

dans laquelle Ar a les significations indiquées en référence à la formule générale I et B–O– représente un groupe éliminable phénoxy, phénylalcoxy ou alcoxy, avec une amine de formule V de la revendication 11, et le cas échéant, on convertit en les sels.

14. Procédé de préparation de sels formés par addition des composés de formule générale I selon l'une des revendications 10 à 13, caractérisé en ce que l'on fait réagir une sulfonylurée de formule générale I avec une amine, un hydroxyde de métal alcalin ou alcalino-terreux ou une base d'ammonium quaternaire.

15. Un produit herbicide et inhibiteur de la croissance des végétaux, caractérisé en ce qu'il contient en tant que substance active au moins une N-(cyclopropyl-triazinyl- ou -pyrimidinyl)-N'-(arylsulfonyl)-urée de formule générale I selon la revendication 1, avec des véhicules et/ou d'autres additifs.

16. L'utilisation des N-(cyclopropyl-triazinyl- ou -pyrimidinyl)-N'-(arylsulfonyl)-urées de formule générale I selon la revendication 1 ou de produits en contenant dans la lutte contre les croissances de végétaux indésirables.

17. L'utilisation des N-(cyclopropyl-triazinyl- et -pyrimidinyl)-N'-(arylsulfonyl)-urées de formule générale I selon la revendication 1 ou de produits en contenant pour l'inhibition de la croissance des végétaux.

18. L'utilisation des N-(cyclopropyl-triazinyl- et -pyrimidinyl)-N'-(arylsulfonyl)-urées de formule générale I selon la revendication 1 ou de produits en contenant pour la lutte sélective en pré-levée ou en post-levée contre les végétaux adventices dans les cultures de végétaux utiles.

19. L'utilisation des N-(cyclopropyl-triazinyl- et -pyrimidinyl)-N'-(arylsulfonyl)-urées de formule générale I selon la revendication 1 ou de produits en contenant pour l'inhibition de la croissance des végétaux au-delà du stade 2 feuilles, caractérisée en ce que l'on applique les substances actives en pré-levée.

20. 2-amino-4-cyclopropyl-pyrimidines de formule V

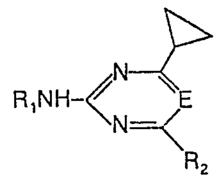

(V),

dans laquelle E, $R_1$ et $R_2$ ont les significations indiquées en référence à la formule générale I, sous réserve toutefois que $R_2$ ne peut représenter Cl, OH ou $OCH_3$, en tant que produits intermédiaires de la préparation des urées de formule générale I.

21. 4-cyclopropyl-1,3,5-triazines de formule VIII

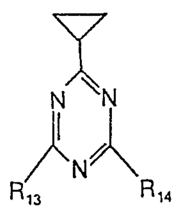

dans laquelle $R_{13}$ représente un groupe trichlorométhyle ou $–NH–R_1$, et $R_{14}$ représente un halogène, un groupe halogénoalkyle en $C_1$–$C_3$, allyle en $C_1$–$C_4$ ou alcoxy en $C_1$–$C_4$, en tant que produits intermédiaires de la préparation des urées de formule générale I.

22. La 2-amino-4-cyclopropyl-6-méthoxy-1,3,5-triazine
selon la revendication 21.

23. La 2-amino-4-cyclopropyl-6-éthoxy-1,3,5-triazine
selon la revendication 21.

24. La 2-amino-4-cyclopropyl-6-trichlorométhyl-1,3,5-triazine
selon la revendication 21.

25. La 2-cyclopropyl-4,6-bis-(trichlorométhyl)-1,3,5-triazine
selon la revendication 21.

26. La N-(4-cyclopropyl-6-méthylthio-1,3,5-triazine-2-yl)-N'-(chlorophénylsulfonyl)-urée,

la N-(4-cyclopropyl-6-méthylthiopyrimidine-2-yl)-N'-(2-méthoxycarbonylphénylsulfonyl)-urée,

la N-(4-cyclopropyl-6-méthylthiopyrimidine-2-yl)-N'-(2-nitrophénylsulfonyl)-urée,

la N-(4-cyclopropyl-6-méthylthiopyrimidine-2-yl)-N'-[2-(2-chloréthoxy)-phénylsulfonyl]-urée ou

la N-(4-cyclopropyl-6-méthylthiopyrimidine-2-yl)-N'-[2-(1,2-dichloréthényloxy-phénylsulfonyl]-urée.

27. Un produit herbicide ou inhibiteur de la croissance des végétaux, caractérisé en ce qu'il contient en tant que substance active, avec des véhicules et/ou d'autres additifs, au moins une N-(cyclopropyl-triazinyl- ou -pyrimidinyl)-N'-(arylsulfonyl)-urée selon la revendication 26.

28. Procédé de préparation:

la N-(4-cyclopropyl-6-méthylthio-1,3,5-triazine-2-yl)-N'-(chlorophénylsulfonyl)-urée,

la N-(4-cyclopropyl-6-méthylthiopyrimidine-2-yl)-N'-(2-méthoxycarbonylphénylsulfonyl)-urée,

la N-(4-cyclopropyl-6-méthylthiopyrimidine-2-yl)-N'-(2-nitrophénylsulfonyl)-urée,

la N-(4-cyclopropyl-6-méthylthiopyrimidine-2-yl)-N'-[2-(2-chloréthoxy)-phénylsulfonyl]-urée ou de

la N-(4-cyclopropyl-6-méthylthiopyrimidine-2-yl)-N'-[2-(1,2-dichloréthényloxy)-phénylsulfonyl]-urée,

caractérisé en ce que l'on fait réagir un phénylsulfonylisocyanate de formule IVa

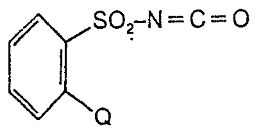

(IVa)

dans laquelle Q représente le chlore, un groupe méthoxycarbonyle, nitro, 2-chloréthoxy ou 1,2-dichloréthényloxy, avec un composé de formule Va

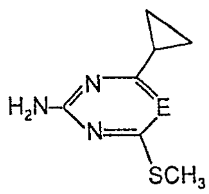

(Va)

dans laquelle E représente N ou CH.

29. L'utilisation de la

N-(4-cyclopropyl-6-méthylthio-1,3,5-triazine-2-yl)-N'-(chlorophénylsulfonyl)-urée,
la N-(4-cyclopropyl-6-méthylthiopyrimidine-2-yl)-N'-(2-méthoxycarbonylphénylsulfonyl)-urée,
la N-(4-cyclopropyl-6-méthylthiopyrimidine-2-yl)-N'-(2-nitrophénylsulfonyl)-urée,
la N-(4-cyclopropyl-6-méthylthiopyrimidine-2-yl)-N'-[2-(2-chloréthoxy)-phénylsulfonyl]-urée ou
la N-(4-cyclopropyl-6-méthylthiopyrimidine-2-yl)-N'-[2-(1,2-dichloréthényloxy)-phénylsulfonyl]-urée,

ou de produits en contenant, dans la lutte contre les croissances de végétaux indésirables.